(19) Europäisches Patentamt
European Patent Office
Office européen des brevets

(11) EP 2 207 522 B1

# (12) EUROPEAN PATENT SPECIFICATION

(45) Date of publication and mention of the grant of the patent:
**06.11.2019 Bulletin 2019/45**

(21) Application number: **08804849.1**

(22) Date of filing: **29.09.2008**

(51) Int Cl.:
*A61K 8/34* (2006.01) *A61K 8/37* (2006.01)
*A61K 8/42* (2006.01) *A61K 8/46* (2006.01)
*A61K 8/49* (2006.01) *A61Q 1/02* (2006.01)
*A61Q 1/06* (2006.01) *A61Q 1/08* (2006.01)
*A61Q 1/10* (2006.01) *A61Q 1/12* (2006.01)
*A61Q 3/02* (2006.01) *A61Q 5/02* (2006.01)
*A61Q 9/04* (2006.01) *A61Q 13/00* (2006.01)
*A61Q 15/00* (2006.01) *A61Q 17/04* (2006.01)
*A61Q 19/00* (2006.01) *A61Q 19/02* (2006.01)
*A61Q 5/06* (2006.01)

(86) International application number:
**PCT/EP2008/062987**

(87) International publication number:
**WO 2009/047150 (16.04.2009 Gazette 2009/16)**

## (54) PERSONAL CARE AND HOUSEHOLD PRODUCT COMPOSITIONS COMPRISING SPECIFIC SULFUR CONTAINING STABILISER COMPOUNDS

KÖRPERPFLEGE- UND HAUSHALTSPRODUKTE UND ZUSAMMENSETZUNGEN MIT SPEZIFISCHEN SCHWEFELHALTIGEN VERBINDUNGEN

PRODUITS DE SOIN PERSONEL ET PRODUITS D'ENTRETIEN MÉNAGER COMPRENANT DES COMPOSÉS STABILISANTS SPECIFIQUES CONTENANT DU SOUFRE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **09.10.2007 US 998162 P**

(43) Date of publication of application:
**21.07.2010 Bulletin 2010/29**

(73) Proprietor: **BASF SE**
**67056 Ludwigshafen am Rhein (DE)**

(72) Inventors:
- **LUPIA, Joseph Anthony**
**Monroe, NY 10950 (US)**
- **REICH, Oliver**
**79639 Grenzach-Wyhlen (DE)**

(74) Representative: **BASF IP Association**
**BASF SE**
**G-FLP-C006**
**67056 Ludwigshafen (DE)**

(56) References cited:
**EP-A2- 0 263 524** **EP-A2- 0 415 887**
**WO-A1-00/25730** **WO-A1-00/25731**
**WO-A1-97/27839** **WO-A2-94/03149**
**WO-A2-2004/100889** **AU-A1- 2008 201 924**
**DE-A1- 1 492 001** **DE-A1- 1 901 935**
**FR-A1- 2 735 977** **GB-A- 2 286 774**
**US-A- 3 262 910** **US-A- 5 688 995**
**US-A- 5 723 435** **US-A1- 2003 149 097**
**US-A1- 2005 019 281**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 207 522 B1

## Description

The present invention relates to the use of certain sulfur containing compounds for the protection of body care products, household products, textiles and fabrics against the deleterious effects of light, heat and oxygen.

The stabilized compositions, for example, comprise dyes that are stabilized against color change. Additionally, the stabilized compositions, for example, are colorless or white and the compositions are stabilized against undesirable color formation.

### Background

WO 00/25730 and WO 00/25731 are aimed at the stabilization of body care and household products.

U.S. Pat. app. No. 60/377,381, filed 05/02/2002, discloses the use of selected hindered nitroxyl, hydroxylamine and hydroxylamine salt compounds in formulations of body care products, household products, textiles and fabrics.

U.S. Pat. app. No. 60/603,590, filed 08/23/2004, discloses the use of selected hindered nitroxyl, hydroxylamine and hydroxylamine salt compounds in formulations of body care products, household products, textiles and fabrics.

WO 01/07550 teaches the treatment of fabric with hindered amine stabilizers.

US 6,254,724 teaches the stabilization of pulp and paper with hindered-amine based compounds.

WO 2003/103622 teaches stabilized body care compositions.

WO 2006/066987 discloses antiradical agents.

US 2007/0196290 discloses UV filters in powder form.

US 2007/0196289 discloses the use of Licochalcone A or an extract of Licochalcone A.

US 7,264,795 discloses various sunscreen compositions .

US 6,919,454 discloses biphenyl-substituted triazines.

US 2007/0218019 discloses surface modified metal oxides, and use thereof in cosmetic preparations.

US 2007/243147 discloses skin and/or hair compositions containing compounds for increased tanning of the skin.

US 2007/243146 discloses vitamin K1 as an energizer in cosmetic formulations.

US 2007/243145 discloses surface-modified metal oxide and their use in cosmetic preparations.

It is now found that certain sulphur-containing compounds provide outstanding protection against light-induced and oxidative discoloration of home personal care products.

### Detailed Disclosure

The present invention pertains to a stabilized composition comprising

(a) a body care product, household product, textile or fabric,
(b) an effective stabilizing amount of at least one compound selected from the group consisting of dilauryl thiodipropionate, distearyl thiodipropionate, dilauryl dithiodipropionate and distearyl dithiodipropionate, and
(c) one or more antioxidant(s) selected from the group consisting of formulae (1), (2), (3) and (4)

(1) 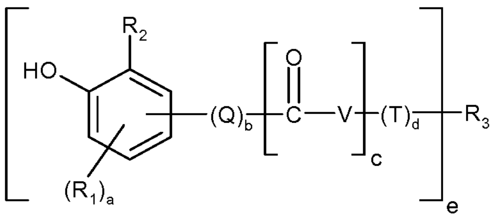 ;

(2) 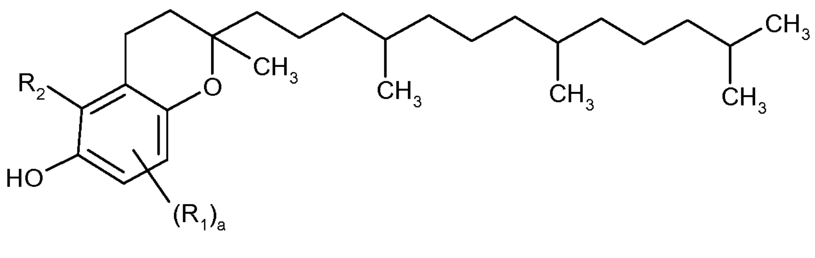 ;

(3) 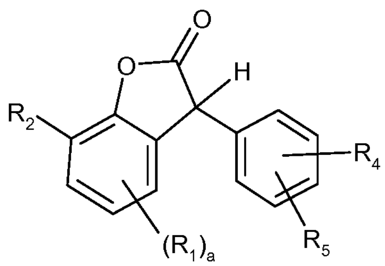 and (4) 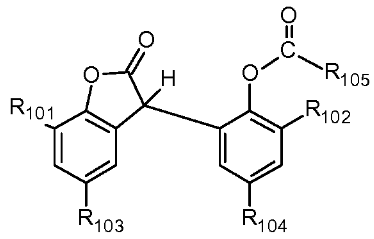 ;

wherein in formulae (1), (2), (3) and (4)

$R_1$ is hydrogen; $C_1$-$C_{22}$alkyl; $C_1$-$C_{22}$alkylthio; $C_5$-$C_7$cycloalkyl; phenyl; $C_7$-$C_9$phenylalkyl; or $SO_3M$;
$R_2$ is $C_1$-$C_{22}$alkyl; $C_5$-$C_7$cycloalkyl; phenyl; or $C_7$-$C_9$phenylalkyl;
Q is $-C_mH_{2m}-$;

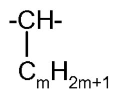 ;

$-C_mH_{2m}-NH$; a radical of formula (1a)

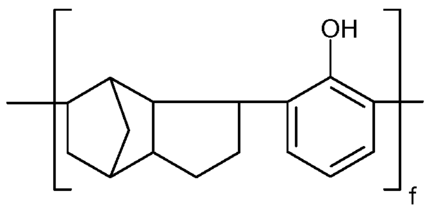 ;or (1b) 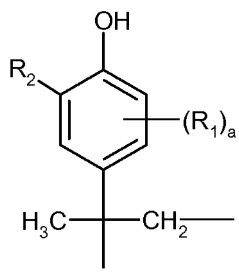 ;

T is $-C_nH_{2n}-$; $-(CH_2)_n-O-CH_2-$;

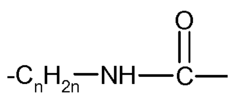 ;

or a radical of formula

(1c) 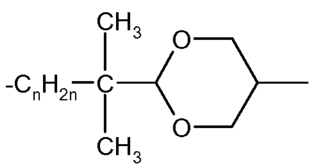 ;

V is -O-; or -NH-;
a is 0; 1; or 2;
b, c and d are each independently of one another 0; or 1;
e is an integer from 1 to 4;
f is an integer from 1 to 3; and
m, n and p are each independently of one another an integer from 1 to 3;
g is 0, 1, 2, or 3;
if e = 1, then
$R_3$ is M; hydrogen; $C_1$-$C_{22}$alkyl; $C_5$-$C_7$cycloalkyl; $C_1$-$C_{22}$alkylthio; $C_2$-$C_{18}$alkenyl; $C_1$-$C_{18}$-phenylalkyl; a radical of formula

(1d) 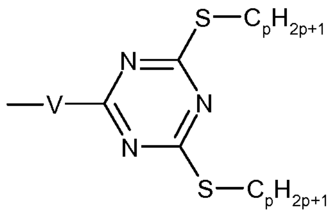 ;

(1e) 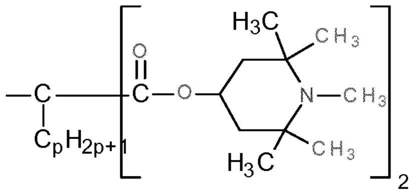 ; or (1f) 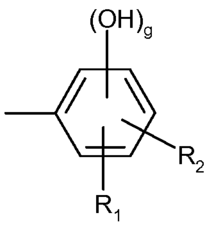

M is alkali; ammonium;
if e = 2, then
$R_3$ is a direct bond; $-CH_2-$;

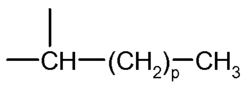 ;

-O-; or -S-;
if
e = 3, then
$R_3$ is the radical of formula

(1g) 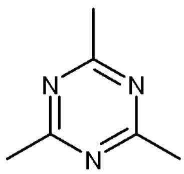 ; (1h) 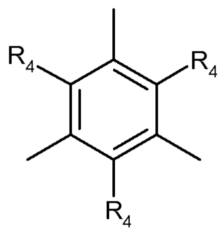 ;

(1i) 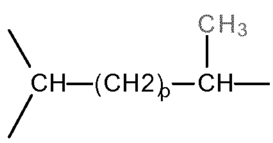 ; or (1k) 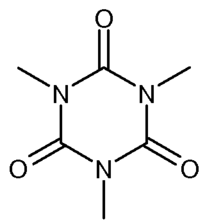 ;

if
e = 4, then
$R_3$ is

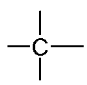 ;

or a direct bond;
$R_4$ and $R_5$ are each independently of the other hydrogen; or $C_1$-$C_{22}$alkyl;

$R_{101}$ and $R_{102}$ are each independently of one another hydrogen; or $C_1$-$C_8$alkyl;
$R_{103}$ and $R_{104}$ are each independently of one another $C_1$-$C_{12}$alkyl; and
$R_{105}$ is $C_1$-$C_7$alkyl.

The term "effective amount" means for example the amount necessary to achieve the desired effect.

Any group denoted as aryl mainly means $C_6$-$C_{12}$aryl; for example, aryl is phenyl or naphthyl; for instance, aryl is phenyl.

Group denoted as alkyl are, within the definitions given, mainly $C_1$-$C_{18}$alkyl, for example methyl, ethyl, propyl such as n- or isopropyl, butyl such as n-, iso-, sec- and tert-butyl, pentyl, hexyl, heptyl, octyl, nonyl, decyl, undecyl, dodecyl, tridecyl, tetradecyl, pentadecyl, hexadecyl, heptadecyl, or octadecyl.

Groups denoted as alkylene are, within the definitions given, for example methylene, 1,2-ethylene, 1,1-ethylene, 1,3-propylene, 1,2-propylene, 1,1-propylene, 2,2-propylene, 1,4-butylene, 1,3-butylene, 1,2-butylene, 1,1-butylene, 2,2-butylene, 2,3-butylene, or $-C_5H_{10}-$, $-C_6H_{12}-$, $C_7H_{14}$, $-C_8H_{16}-$, $-C_9H_{18}-$, $-C_{10}H_{20}-$, $-C_{11}H_{22}-$, $-C_{12}H_{24}-$, $-C_{13}H_{26}-$, $-C_{14}H_{28}-$, $-C_{15}H_{30}-$, $-C_{16}H_{32}-$, $-C_{17}H_{34}-$, or $-C_{18}H_{36}-$.

Groups denoted as cycloalkyl or cycloalkoxy are mainly $C_5$-$C_{12}$cycloalkyl or $C_5$-$C_{12}$cycloalkoxy, the cycloalkyl part being, for example, cyclopentyl, cyclohexyl, cycloheptyl, cyclooctyl, cyclononyl, cyclodecyl, cycloundecyl, or cyclododecyl. Cycloalkenyl is mainly $C_5$-$C_{12}$cycloalkenyl including cyclopentenyl, cyclohexenyl, cycloheptenyl, cyclooctenyl, cyclononenyl, cyclodecenyl, cycloundecenyl, or cyclododecenyl.

Aralkyl or aralkoxy is, for example, phenylalkyl or phenylalkoxy, which is alkyl or alkoxy substituted by phenyl. Examples for phenylalkyl or phenylalkoxy are, within the definitions given, benzyl, benzyloxy, alpha-methylbenzyl, alpha-methylbenzyloxy, cumyl, or cumyloxy.

Alkenyl residues are mainly alkenyl of 2 to 18 carbon atoms; for example, allyl.

Alkynyl residues are mainly alkynyl of 2 to 12 carbon atoms; for example, propargyl.

A group denoted as acyl is mainly R(C=O)-, where R is an aliphatic or aromatic moiety.

An aliphatic or aromatic moiety, such as mentioned above or other definitions, mainly is an aliphatic or aromatic $C_1$-$C_{30}$hydrocarbon; examples are aryl, alkyl, cycloalkyl, alkenyl, cycloalkenyl, bicycloalkyl, bicycloalkenyl, and combinations of these groups.

Examples for acyl groups are alkanoyl of 2 to 12 carbon atoms, alkenoyl of 3 to 12 carbon atoms, or benzoyl.

Alkanoyl embraces, for example, formyl, acetyl, propionyl, butyryl, pentanoyl, oroctanoyl; for example, $C_2$-$C_8$alkanoyl; for instance, acetyl.

Alkenoyl residues are, for example, acryloyl, or methacryloyl.

The alkyl groups in the different substituents may be linear or branched.

Examples for alkenyl groups with 2 to 4 carbon atoms are ethenyl, propenyl, or butenyl. Examples for alkyl groups with 1 to 4 carbon atoms interrupted by one or two oxygen atoms are $-CH_2-O-CH_3$, $-CH_2-CH_2-O-CH_3$, $-CH_2-CH_2-O-CH_2-CH_3$, $-CH_2-O-CH_2-CH_2-O-CH_3$, or $-CH_2-O-CH_2-O-CH_3$.

Examples for hydroxy substituted alkyl groups with 2 to 6 carbon atoms are hydroxy ethyl, di-hydroxy ethyl, hydroxy propyl, di-hydroxy propyl, hydroxy butyl, hydroxy pentyl, or hydroxy hexyl.

The present compositions may comprise further traditional additives, for example ultraviolet (UV) light absorbers and hindered amine light stabilizers.

The present invention pertains to a stabilized composition comprising

(a) a body care product, household product, textile or fabric,
(b) an effective stabilizing amount of one or more compounds selected from the group consisting of dilauryl thiodipropionate, distearyl thiodipropionate, dilauryl dithiodipropionate and distearyl dithiodipropionate,
and
(d) one or more compounds selected from the group consisting of the ultraviolet light absorbers, hindered amine light stabilizers, complex formers, optical brighteners, surfactants and polyorganosiloxanes.

The additional additives of present component (d) are for example those disclosed in copending U.S. application Nos. 09/830,788, filed May 1, 2001 and 09/830,787, filed May 1, 2001, published as WO 00/25730 and WO 00/25731.

The UV absorbers of component (d) are, for example, selected from group consisting of 2H-benzotriazoles, s-triazines, benzophenones, alpha-cyanoacrylates, oxanilides, benzoxazinones, benzoates and alpha-alkyl cinnamates.

The UV absorbers of component (d) are, for example:
2,4,6-tris(2-hydroxy-4-octyloxyphenyl)-1,3,5-triazine; 2-(2,4-dihydroxyphenyl)-4,6-bis(2,4-dimethylphenyl)-1,3,5-triazine; 2,4-bis(2-hydroxy-4-propyloxyphenyl)-6-(2,4-dimethylphenyl)-1,3,5-triazine; 2-(2-hydroxy-4-octyloxyphenyl)-4,6-bis(4-methylphenyl)-1,3,5-triazine; 2-(2-hydroxy-4-dodecyloxyphenyl)-4,6-bis(2,4-dimethylphenyl)-1,3,5-triazine; 2-[2-hydroxy-4-(2-hydroxy-3-butyloxypropyloxy)phenyl]-4,6-bis(2,4-dimethylphenyl)-1,3,5-triazine; 2-[2-hydroxy-4-(2-hydroxy-3-octyloxypropyloxy)phenyl]-4,6-bis(2,4-dimethylphenyl)-1,3,5-triazine; 2-[2-hydroxy-4-(2-hydroxy-3-tridecyloxy-propyloxy)phenyl]-4,6-bis(2,4-dimethylphenyl)-1,3,5-triazine; 5-chloro-2-(2-hydroxy-3,5-di-tert-butylphenyl)-2H-benzotriazole; 2-(2-hydroxy-3-dodecyl-5-methylphenyl)-2H-benzotriazole; 5-chloro-2-(2-hydroxy-3-tert-butyl-5-methylphenyl)-2H-benzotriazole; bis-(3-(2H-benzotriazol-2-yl)-2-hydroxy-5-tert-octyl)methane; 2-(2-hydroxy-3,5-di-tert-butylphenyl)-2H-benzotriazole; 2-(2-hydroxy-3,5-di-tert-amylphenyl)-2H-benzotriazole; 2-(2-hydroxy-3,5-di-alpha-cumylphenyl)-2H-benzotriazole; 2-(2-hydroxy-3-alpha-cumyl-5-tert-octylphenyl)-2H-benzotriazole; 2-(2-hydroxy-5-tert-octylphenyl)-2H-benzotriazole; 3-(2H-benzotriazol-2-yl)-4-hydroxy-5-(1-methylpropyl)-benzenesulfonic acid monosodium salt; 3-tert-butyl-4-hydroxy-5-(2H-benzotriazol-2-yl)-hydrocinnamic acid and sodium salt; 12-hydroxy-3,6,9-trioxadodecyl 3-tert-butyl-4-hydroxy-5-(2H-benzotriazol-2-yl)-hydrocinnamate; octyl 3-tert-butyl-4-hydroxy-5-(2H-benzotriazol-2-yl)-hydrocinnamate; 4,6-bis(2,4-dimethylphenyl)-2-(4-(3-dodecyloxy*-2-hydroxypropoxy)-2-hydroxyphenyl)-s-triazine (*is mixture of $C_{12-14}$oxy isomers); 4,6-bis(2,4-dimethylphenyl)-2-(4-octyloxy-2-hydroxyphenyl)-s-triazine; 2,4-dihydroxybenzophenone; 2,2'-dihydroxy-4,4'-dimethoxy-5,5'-disulfobenzophenone, disodium salt; 2-hydroxy-4-octyloxybenzophenone; 2-hydroxy-4-dodecyloxybenzophenone; 2,4-dihydroxybenzophenone; 2,2',4,4'-tetrahydroxybenzophenone; 4-aminobenzoic acid; 2,3-dihydroxypropyl-4-aminobenzoic acid; 3-(4-imidazolyl)acrylic acid; 2-phenyl-5-benzimidazole sulfonic acid; N,N,N-trimethyl-alpha-(2-oxo-3-bornylidene)-p-toluidinium methyl sulfate; 5-benzoyl-4-hydroxy-2-methoxybenzenesulfonic acid, sodium salt; 3-(4-benzoyl-3-hydroxy-phenoxy)-2-hydroxy-N,N,N- trimethyl-1-propanaminium chloride; 3-[4-(2H- benzotriazol-2-yl)-3-hydroxyphenoxy]- 2-hydroxy-N,N,N-trimethyl-1-propanaminium, chloride; 2-(2-hydroxy-5-methylphenyl)-2H-benzotriazole; or 2,2'-dihydroxy-4,4'-dimethoxybenzophenone (Uvinul® 3049).

For instance, suitable UV absorbers are selected from the group consisting of 3-(2H-benzotriazol-2-yl)-4-hydroxy-5-(1-methylpropyl)-benzenesulfonic acid monosodium salt; 3-tert-butyl-4-hydroxy-5-(2H-benzotriazol-2-yl)-hydrocinnamic acid and sodium salt; 2-(2-hydroxy-3,5-di-tert-butylphenyl)-2H-benzotriazole; 2-(2-hydroxy-3,5-di-tert-amylphenyl)-2H-benzotriazole; 4,6-bis(2,4-dimethylphenyl)-2-(4-(3-dodecyloxy*-2-hydroxypropoxy)-2-hydroxyphenyl)-s-triazine (*is mixture of $C_{12-14}$oxy isomers); 12-hydroxy-3,6,9-trioxadodecyl 3-tert-butyl-4-hydroxy-5-(2H-benzotriazol-2-yl)-hydrocinnamate; 2,4-dihydroxybenzophenone; 2,2'-dihydroxy-4,4'-dimethoxy-5,5'-disulfobenzophenone, disodium salt; 2,2',4,4'-tetrahydroxybenzophenone; 3-(4-benzoyl-3-hydroxyphenoxy)-2-hydroxy-N,N,N-trimethyl-1-propanaminium chloride; 3-[4-(2H- benzotriazol-2-yl)-3-hydroxyphenoxy]-2-hydroxy-N,N,N-trimethyl-1-propanaminium, chloride; 5-benzoyl-4-hydroxy-2-methoxy-benzenesulfonic acid, sodium salt; and 2-(2-hydroxy-3-alpha-cumyl-5-tert-octylphenyl)-2H-benzotriazole.

Additional suitable antioxidants of component (c) are for example selected from the hindered phenolic and benzofuranone stabilizers.

Suitable antioxidants of component (c) are, for example, selected from the group consisting of

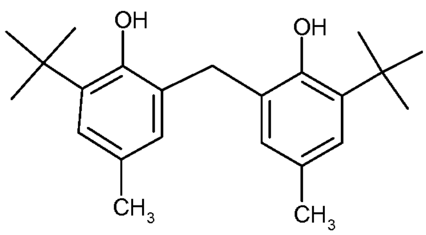

,

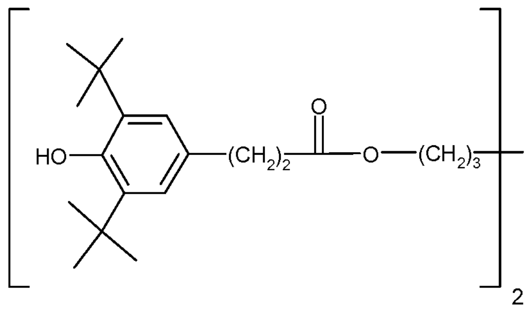

,

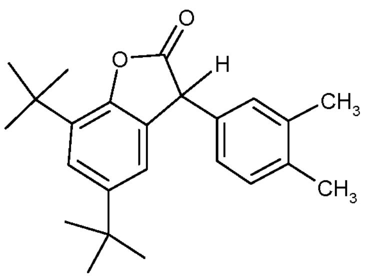

,

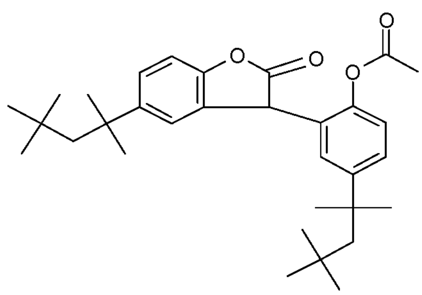

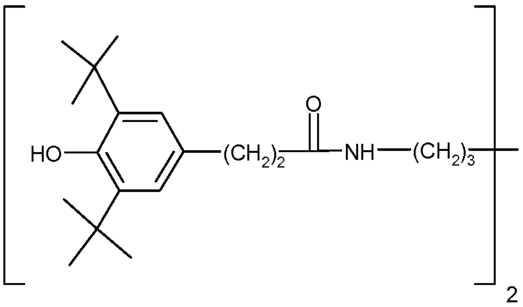
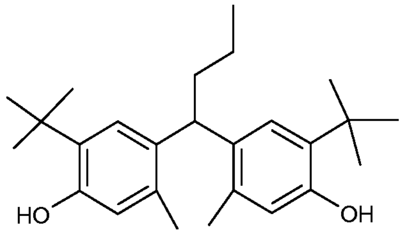
,

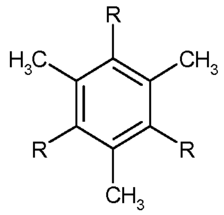
R =
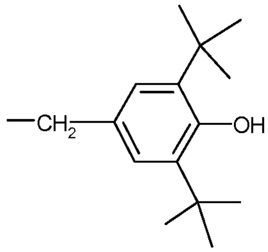
,

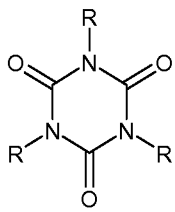
R =
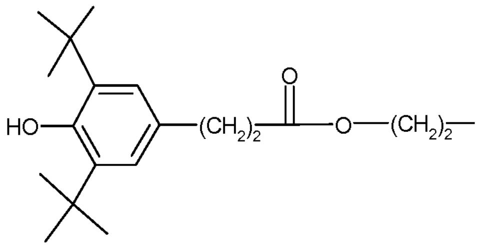
,

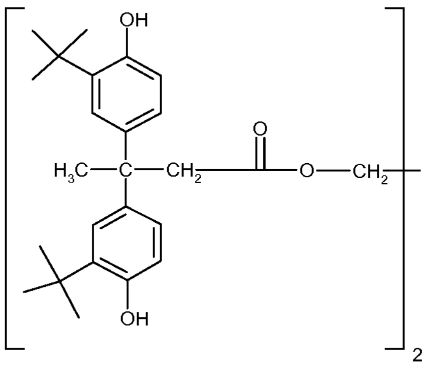
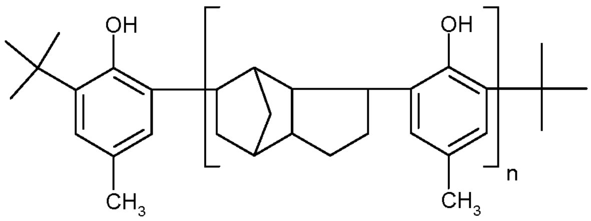
n = 1-3
,

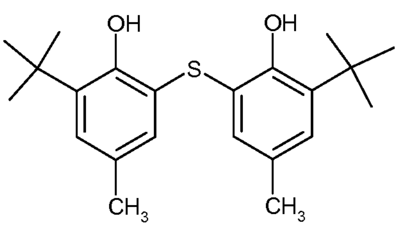
,
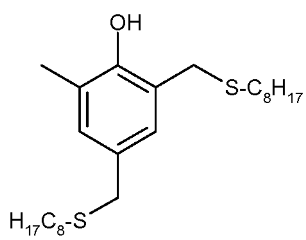
,

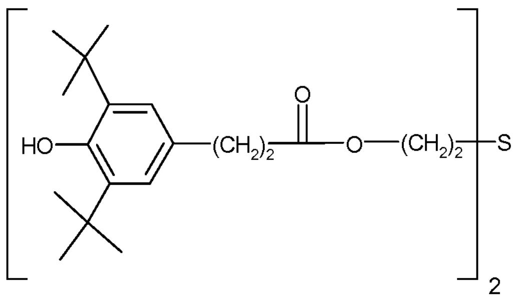

,

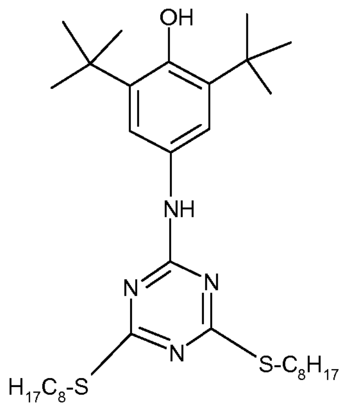

,

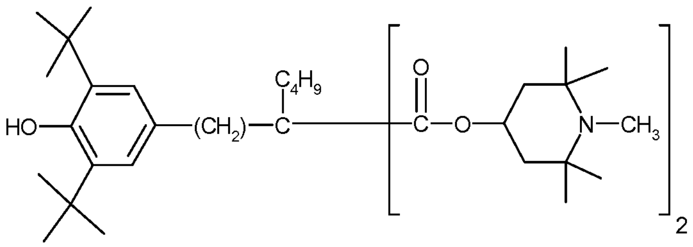

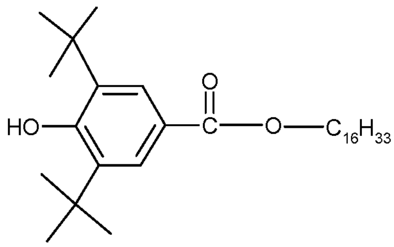

,

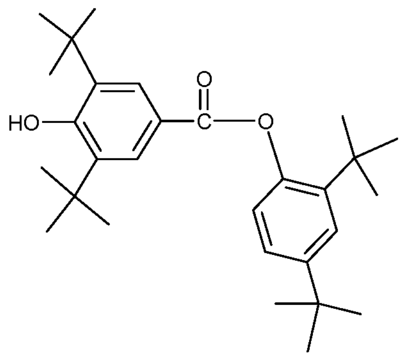

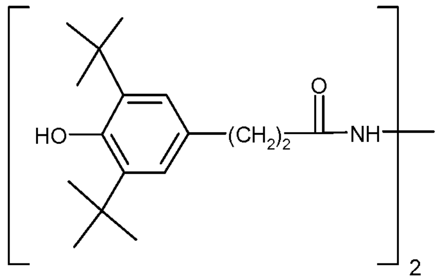

,

,

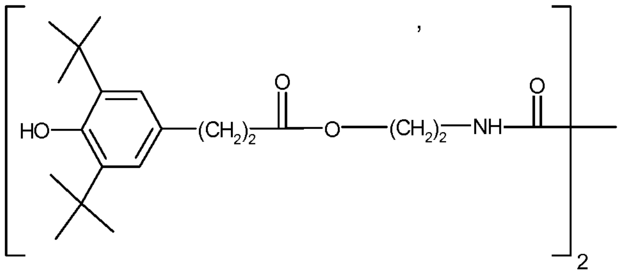

,

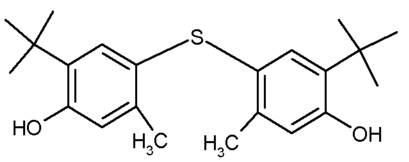

,

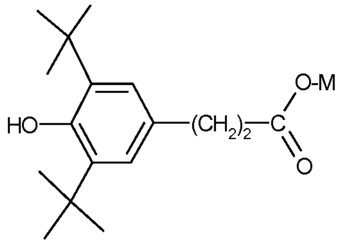

M = H, ammonium, alkali

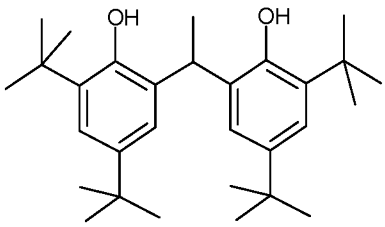

,

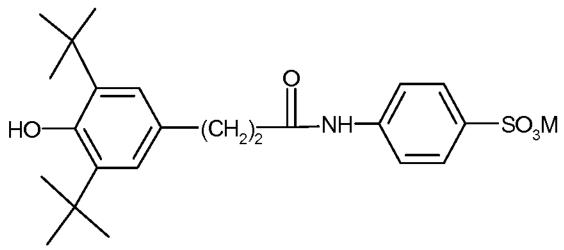

M = H, Na

,

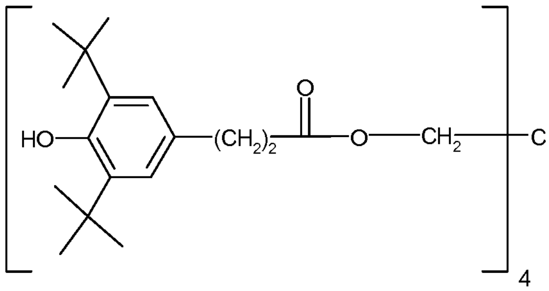

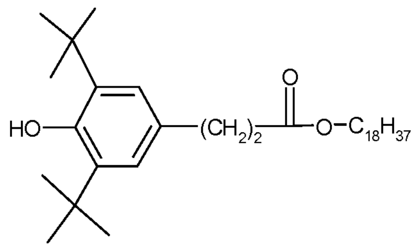

,

and

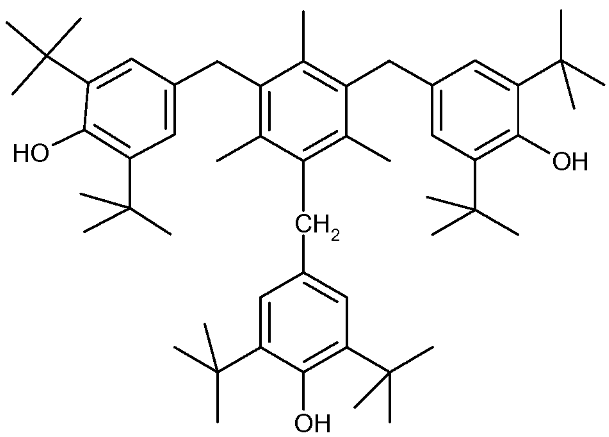

.

The hindered amine light stabilizers (HALS) of component (d) are for example known commercial compounds. They are, for example, selected from the group consisting of bis(2,2,6,6-tetramethylpiperidin-4-yl)sebacate, bis(2,2,6,6-tetramethylpiperidin-4-yl)succinate, bis(1,2,2,6,6-pentamethylpiperidin-4-yl)sebacate, n-butyl-3,5-di-tert-butyl-4-hydroxybenzyl-malonic acid-bis(1,2, 2,6,6-pentamethylpiperidyl)ester, the condensate of 1-hydroxyethyl-2,2,6,6-tetramethyl-4-hydroxypiperidine and succinic acid, the condensate of N,N'-bis-(2,2,6,6-tetramethyl-4-piperidyl)hexamethylenediamine and 4-tert-octylamino-2,6-dichloro-1,3,5-s-triazine, tris(2,2,6,6-tetramethyl-4-piperidyl)nitrilotriacetate, tetrakis(2,2,6,6-tetramethyl-4-piperidyl)-1,2,3,4-butanetetraoate, 1,1'-(1,2-ethanediyl)-bis(3,3,5,5-tetramethylpiperazinone), 4-benzoyl-2,2,6,6-tetramethylpiperidine, 4-stearyloxy-2,2,6,6-tetramethyl-piperidine, bis(1,2,2,6,6-pentamethylpiperidyl)-2-n-butyl-2-(2-hydroxy-3,5-di-tert-butylbenzyl)malonate, 3-n-octyl-7,7,9,9-tetramethyl-1,3,8-triazaspiro[4.5]decane-2,4-dione, the condensate of N,N-bis(2,2,6,6-tetramethyl-4-piperidyl)hexamethylenediamine and 4-morpholino-2,6-dichloro-1,3,5-triazine, the condensate of 2-chloro-4,6-di(4-n-butylamino-2,2,6,6-tetramethylpiperidyl)-1,3,5-triazine and 1,2-bis(3-aminopropylamino)ethane, the condensate of 2-chloro-4,6-di(4-n-butylamino-1,2,2,6,6-pentamethylpiperidyl)-1,3,5-triazine and 1,2-bis(3-aminopropylamino)ethane, 8-acetyl-3-dodecyl-7,7,9,9-tetramethyl-1,3,8-triazaspiro[4.5]-decane-2,4-dione, 3-dodecyl-1-(2,2,6,6-tetramethyl-4-piperidyl)pyrrolidin-2,5-dione, 3-dodecyl-1-(1,2,2,6,6-pentamethyl-4-piperidyl)-pyrrolidine-2,5-dione, a mixture of 4-hexadecyl-oxy- and 4-stearyloxy-2,2,6,6-tetramethylpiperidine, the condensate of N,N'-bis(2,2,6,6-tetramethyl-4-piperidyl)hexamethylenediamine and 4-cyclohexylamino-2,6-dichloro-1,3,5-triazine, the condensate of 1,2-bis(3-aminopropylamino)ethane and 2,4,6-trichloro-1,3,5-triazine and 4-butylamino-2,2,6,6-tetramethylpiperidine (CAS reg. No. [136504-96-6]); (2,2,6,6-tetramethyl-4-piperidyl)-n-dodecylsuccinimide, (1,2,2,6,6-pentamethyl-4-piperidyl)-n-dodecylsuccinimide, 2-undecyl-7,7,9,9-tetramethyl-1-oxa-3,8-diaza-4-oxo-spiro[4,5]decane, the reaction product of 7,7,9,9-tetramethyl-2-cycloundecyl-1-oxa-3,8-diaza-4-oxospiro-[4,5]decane and epichlorohydrin, tetra(2,2,6,6-tetramethylpiperidin-4-yl)-butane-1,2,3,4-tetracarboxylate, tetra(1,2,2,6,6-pentamethylpiperidin-4-yl)-butane-1,2,3,4-tetracarboxylate, 2,2,4,4-tetramethyl-7-oxa-3,20-diaza-21-oxo-dispiro[5.1.11.2] -heneicosan, 8-acetyl-3-dodecyl-1,3,8-triaza-7,7,9,9-tetramethylspiro[4,5] -decane-2,4-dione,

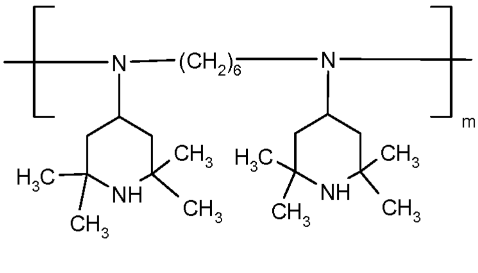,

wherein m is a value from 5-50,

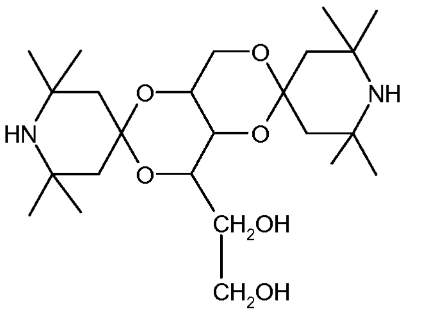,

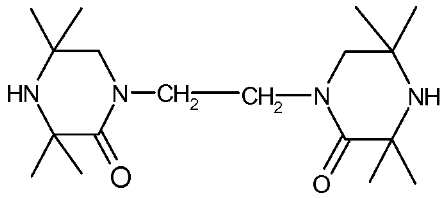,

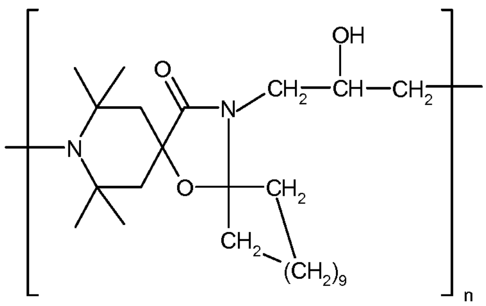,

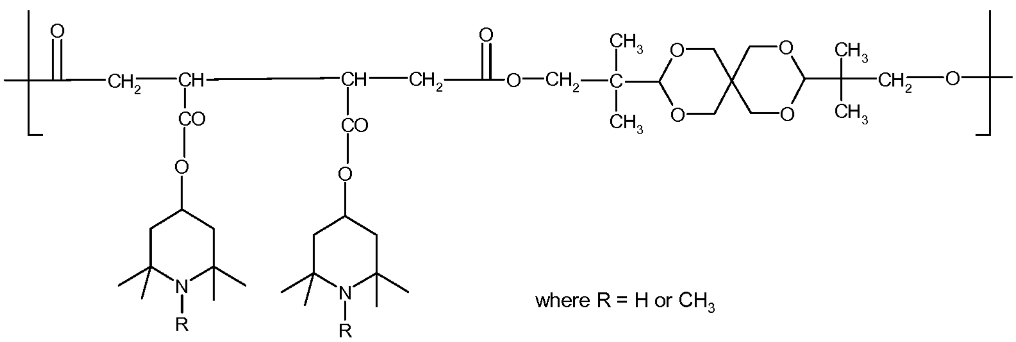

where R = H or $CH_3$

and

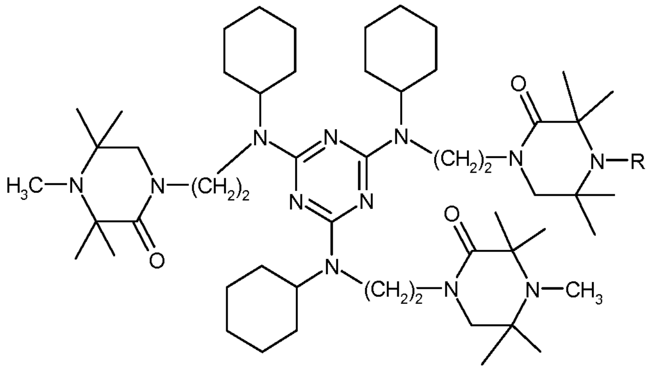

where R = H or $CH_3$

Additional hindered amine light stabilizer of according to the instant invention is a compound of component (d) selected from the group consisting of

(a) Monosodium 1-methoxy-2,2,6,6-tetramethyl-4-acetoxypiperidinium citrate;
(b) Monopotassium 1-methoxy-2,2,6,6-tetramethyl-4-acetamidopiperidinium phosphate;
(c) Monoammonium 1-butoxy-2,2,6,6-tetramethyl-4-acetamidopiperidinium sulfate;
(d) Mono-(N,N,N-triethylammonium) 1-phenoxy-2,2,6,6-tetramethyl-4-methoxy-piperidinium tartrate;
(e) Mono-[N,N-di-n-butyl ammonium] 1-ethoxy-2,2,6,6-tetramethyl-4-acetoxypiperidiniummaleate;
(f) Mono-[N,N,N-(2-hydroxyethyl)ammonium] 1-methoxy-2,2,6,6-tetramethyl-4-propoxy-piperidinium oxalate;
(g) Monosodium 1-methoxy-2,2,6,6-tetramethyl-4-(2-hydroxy-4-oxapentoxy)piperidiniumcarbonate;
(h) Dipotassium 1-(2-hydroxy-2-methylpropoxy) -2,2,6,6-tetramethyl-4-hydroxypiperidiniumcitrate;
(i) Monosodium bis(1-(2-hydroxy-2-methylpropoxy) -2,2,6,6-tetramethyl-4-hydroxypiperidinium) citrate;
(j) Diammonium (1-(2-hydroxy-2-methylpropoxy) -2,2,6,6-tetramethyl-4-hydroxypiperidinium) citrate;
(k) Monopotassium monosodium bis(1-(2-hydroxy-2-methylpropoxy) -2,2,6,6-tetramethyl-4-hydroxypiperidinium) ethylenediaminetetraacetate;
(l) Monosodium tris(1-(2-hydroxy-2-methylpropoxy) -2,2,6,6-tetramethyl-4-acetamidopiperidinium) ethylenediaminetetraacetate;
(m) Monocalcium bis(1-(2-hydroxy-2-methylpropoxy) -2,2,6,6-tetramethyl-4-oxopiperidinium) ethylenediaminetetraacetate;
(n) Monoammonium tetra(1-(2-hydroxy-2-methylpropoxy) -2,2,6,6-tetramethyl-4-hydroxy-piperidinium) diethylenetriaminepentaacetate;
(o) Disodium tris(1-(2-hydroxy-2-methylpropoxy) -2,2,6,6-tetramethyl-4-acetamidopiperidinium) diethylenetriaminepentaacetate;
(p) Tripotassium di(1-(2-hydroxy-2-methylpropoxy) -2,2,6,6-tetramethyl-4-oxopiperidinium)diethylenetriaminepentaacetate;
(q) Monobenzylammonium bis(1-(2-hydroxy-2-methylpropoxy) -2,2,6,6-tetramethyl-4-hydroxypiperidinium) nitrilotriacetate;
(r) Di-[N,N-diethylammonium] mono(1-(2-hydroxy-2-methylpropoxy) -2,2,6,6-tetramethyl-4-acetamidopiperidinium) nitrilotriacetate;
(s) Monosodium bis(1-(2-hydroxy-2-methylpropoxy) -2,2,6,6-tetramethyl-4-oxopiperidinium) nitrilotriacetate;
(t) Diammonium tris(1-(2-hydroxy-2-methylpropoxy) -2,2,6,6-tetramethyl-4-hydroxy-piperidinium) diethylenetriaminepentamethylenephosphonate;
(u) Monosodium tetra(1-(2-hydroxy-2-methylpropoxy) -2,2,6,6-tetramethyl-4-acetamidopiperidinium) diethylenetriaminepentamethylenephosphonate; and,
(v) Tripotassium bis(1-(2-hydroxy-2-methylpropoxy) -2,2,6,6-tetramethyl-4-oxopiperidinium) diethylenetriaminepentamethylenephosphonate.

Additional hindered amine light stabilizer of according to the instant invention is a compound of component (d) selected from the group consisting of

(a) bis(1-octyloxyl-2,2,6,6-tetramethylpiperidin-4-yl) sebacate hydrochloride;
(b) 1-methoxy-2,2,6,6-tetramethyl-4-acetoxypiperidinium citrate;
(c) 1-methoxy-2,2,6,6-tetramethyl-4-acetamidopiperidinium phosphate;
(d) 1-butoxy-2,2,6,6-tetramethyl-4-acetamidopiperidinium bisulfate;

(e) 1-methoxy-2,2,6,6-tetramethyl-4-oxo-piperidinium methyl sulfonate;
(f) 1-methoxy -2,2,6,6-tetramethyl-4-oxo-piperidinium acetate;
(g) 1-phenoxy-2,2,6,6-tetramethyl-4-methoxy-piperidinium tartrate;
(h) 1-benzoxy-2,2,6,6-tetramethyl-4-methoxy-piperidinium acetate;
(i) 1-ethoxy-2,2,6,6-tetramethyl-4-acetoxypiperidinium maleate;
(j) 1-methoxy-2,2,6,6-tetramethyl-4-propoxy-piperidinium mandelate;
(k) 1-methoxy-2,2,6,6-tetramethyl-4-propoxy-piperidinium oxalate;
(l) 1-methoxy-2,2,6,6-tetramethyl-4-(2-hydroxy-4-oxapentoxy)piperidinium bicarbonate;
(m) 1-methoxy-2,2,6,6-tetramethyl-4-(2-hydroxy-4-oxapentoxy)piperidinium glycolate;
(n) 1-methoxy-2,2,6,6-tetramethyl-4-hydroxypiperidinium gluconate;
(o) 1-methoxy-2,2,6,6-tetramethyl-4-hydroxypiperidinium ascorbate;
(p) 1-methoxy-2,2,6,6-tetramethyl-4-hydroxypiperidinium benzene sulfonate;
(q) 1-(2-hydroxy-2-methylpropoxy) -2,2,6,6-tetramethyl-4-hydroxypiperidinium ascorbate;
(r) 1-(2-hydroxy-2-methylpropoxy) -2,2,6,6-tetramethyl-4-hydroxypiperidinium citrate;
(s) bis(1-(2-hydroxy-2-methylpropoxy) -2,2,6,6-tetramethyl-4-hydroxypiperidinium) citrate;
(t) tris(1-(2-hydroxy-2-methylpropoxy) -2,2,6,6-tetramethyl-4-hydroxypiperidinium) citrate;
(u) tetra(1-(2-hydroxy-2-methylpropoxy)-2,2,6,6-tetramethyl-4-hydroxypiperidinium) ethylenediaminetetraacetate;
(v) tetra(1-(2-hydroxy-2-methylpropoxy) -2,2,6,6-tetramethyl-4-acetamidopiperidinium) ethylenediaminetetraacetate;
(w) tetra(1-(2-hydroxy-2-methylpropoxy) -2,2,6,6-tetramethyl-4-oxopiperidinium) ethylenediaminetetraacetate;
(x) penta(1-(2-hydroxy-2-methylpropoxy) -2,2,6,6-tetramethyl-4-hydroxypiperidinium) diethylenetriaminepentaacetate;
(y) penta(1-(2-hydroxy-2-methylpropoxy) -2,2,6,6-tetramethyl-4-acetamidopiperidinium) diethylenetriaminepentaacetate;
(z) penta(1-(2-hydroxy-2-methylpropoxy) -2,2,6,6-tetramethyl-4-oxopiperidinium) diethylenetriaminepentaacetate;
(aa) tri(1-(2-hydroxy-2-methylpropoxy) -2,2,6,6-tetramethyl-4-hydroxypiperidinium) nitrilotriacetate;
(bb) tri(1-(2-hydroxy-2-methylpropoxy) -2,2,6,6-tetramethyl-4-acetamidopiperidinium) nitrilotriacetate;
(cc) tri(1-(2-hydroxy-2-methylpropoxy) -2,2,6,6-tetramethyl-4-oxopiperidinium) nitrilotriacetate;
(dd) penta(1-(2-hydroxy-2-methylpropoxy)-2,2,6,6-tetramethyl-4-hydroxypiperidinium) diethylenetriaminepentamethylenephosphonate;
(ee) penta(1-(2-hydroxy-2-methylpropoxy) -2,2,6,6-tetramethyl-4-acetamidopiperidinium) diethylenetriaminepentamethylenephosphonate; and,
(ff) penta(1-(2-hydroxy-2-methylpropoxy) -2,2,6,6-tetramethyl-4-oxopiperidinium) diethylenetriaminepentamethylenephosphonate.

The complex formers of component (d) are for example nitrogen-containing complex formers or polyanionically-derived natural polysaccharides, for example those containing phosphate, phosphonate or methylphosphonate groups, such as chitin derivatives, e.g. sulfochitin, carboxymethylchitin, phosphochitin or chitosan derivatives, for example sulfochitosan, carboxymethylchitosan or phosphochitosan.

The complex formers are for example selected from the group consisting of ethylenediaminetetracetic acid (EDTA), nitrilotriacetic acid (NTA), beta-alaninediacetic acid (EDETA), ethylenediaminedisuccinic acid (EDDS),

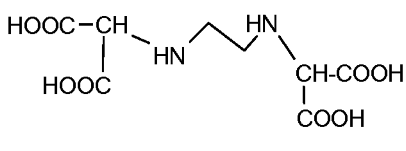

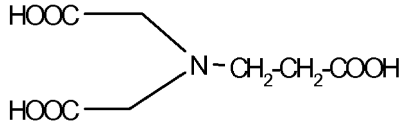

aminetrimethylenephosphoric acid (ATMP) conforming to

formula 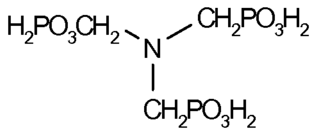 ,

serinediacetic acid (SDA) conforming to formula

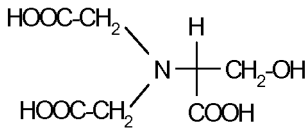 ,

asparaginediacetic acid conforming to formula

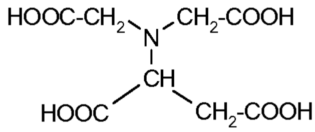

and methylglycinediacetic acid (MGDA) conforming to formula

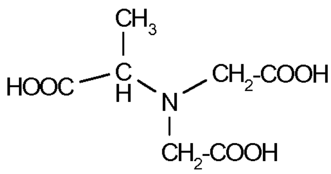 .

The present stabilizer systems are particularly suitable for stabilizing fragrances in body care products or household products. One particular use is for skin-care products, as bath and shower products, preparations containing fragrances and odoriferous substances, hair-care products, dentifrices, deodorizing and antiperspirant preparations, decorative preparations, light protection formulations and preparations containing active ingredients.

Suitable skin-care products are, in particular, body oils, body lotions, body gels, treatment creams, skin protection ointments, shaving preparations, such as shaving foams or gels, skin powders, such as baby powder, moisturising gels, moisturising sprays, revitalising body sprays, cellulite gels and peeling preparations.

Preparations containing fragrances and odoriferous substances are in particular scents, perfumes, toilet waters and shaving lotions (aftershave preparations).

Suitable hair-care products are, for example, shampoos for humans and animals, in particular dogs and cats, hair conditioners, products for styling and treating hair, perming agents, hair sprays and lacquers, hair gels, hair fixatives and hair dyeing or bleaching agents.

Suitable dentifrices are in particular tooth creams, toothpastes, mouthwashes, mouth rinses, anti-plaque preparations and cleaning agents for dentures.

Suitable decorative preparations are in particular lipsticks, nail varnishes, eye shadows, mascaras, dry and moist make-up, rouge, powders, depilatory agents and suntan lotions.

Suitable cosmetic formulations containing active ingredients are in particular hormone preparations, vitamin preparations, vegetable extract preparations and antibacterial preparations.

The present body care products can be in the form of creams, ointments, pastes, foams, gels, lotions, powders, make-ups, sprays, sticks or aerosols. The present stabilizer systems may be present in the oil phase or in the aqueous or aqueous/alcoholic phase.

The compounds of component (b) are present, for example, in the body care and household products in a concentration of about 5 to about 10000 ppm, based on the total formulation by weight, for example from about 10 to about 5000 ppm, for example from about 100 to about 5000 ppm. For example, the compounds of component (b) are present in the body care and household products in a concentration of about 5, 10, 15, 20, 25, 35, 40, 45 or 50 ppm, based on the total formulation by weight. For example, the compounds of component (b) are present from about 5 to about 5000 ppm in the formulations (compositions) of this invention.

The antioxidants of component (c) are present, for example, in the body care and household products in a concentration of about 5 to about 10000 ppm, based on the total formulation by weight, for example from about 10 to about 5000 ppm, for example from about 100 to about 5000 ppm. For example the antioxidants of component (c) are present in the body care and household products in a concentration of about 5, 10, 15, 20, 25, 35, 40, 45 or 50 ppm, based on the total formulation by weight. For example, the antioxidants of component (c) are present from about 5 to about 5000 ppm in the formulations or compositions of this invention.

The additives of component (d) are present, for example, in the body care and household products in a concentration of about 5 to about 10000 ppm, based on the total formulation by weight, for example from about 10 to about 5000 ppm, for example from about 100 to about 5000 ppm. For example, the additives of component (d) are present in the body care and household products in a concentration of about 5, 10, 15, 20, 25, 35, 40, 45 or 50 ppm, based on the total formulation by weight. For example, the additives of component (d) are present from about 5 to about 5000 ppm in the formulations or compositions of this invention.

Laundry detergents, fabric softeners or other products, from which the compounds of component (b) are intended for deposition onto fabrics with use, are considered household products of this invention, and the above concentration levels also pertain thereto. The present compounds of component (b) are effective at stabilizing the laundry detergents and fabric softeners, as well as the fabrics treated therewith. They are especially useful in products with high fragrance amounts, e.g. air fresheners or softeners; and for products containing bleach, e.g. peroxide or hypochlorite.

Creams are oil-in-water emulsions containing more than 50% water. The oil-containing base used therein is usually mainly fatty alcohols, for example lauryl, cetyl or stearyl alcohol, fatty acids, for example palmitic or stearic acid, liquid to solid waxes, for example isopropylmyristate or beeswax and/or hydrocarbon compounds, such as paraffin oil. Suitable emulsifiers are surfactants having primarily hydrophilic properties, such as the corresponding nonionic emulsifiers, for example fatty acid esters of polyalcohols of ethylene oxide adducts, such as polyglycerol fatty acid ester or polyoxyethylenesorbitan fatty acid ether (Tween trademarks); polyoxyethylene fatty alcohol ether or their esters or the corresponding ionic emulsifiers, such as the alkali metal salts of fatty alcohol sulfonates, sodium cetyl sulfate or sodium stearyl sulfate, which are usually used together with fatty alcohols, such as cetyl alcohol or stearyl alcohol. In addition, creams contain agents which reduce water loss during evaporation, for example polyalcohols, such as glycerol, sorbitol, propylene glycol, and/or polyethylene glycols.

Ointments are water-in-oil emulsions which contain up to 70%, for instance not more than 20 to 50%, of water or of an aqueous phase. The oil-containing phase contains predominantly hydrocarbons, such as paraffin oil and/or solid paraffin which for instance contains hydroxy compounds, for example fatty alcohol or their esters, such as cetyl alcohol or wool wax for improving the water absorption. Emulsifiers are corresponding lipophilic substances, such as sorbitan fatty acid ester. In addition, the ointments contain moisturisers such as polyalcohols, for example glycerol, propylene glycol, sorbitol and/or polyethylene glycol as well as preservatives.

Rich creams are anhydrous formulations and are produced on the basis of hydrocarbon compounds, such as paraffin, natural or partially synthetic fats, for example coconut fatty acid triglycerides or, for instance, hardened oils and glycerol partial fatty acid esters.

Pastes are creams and ointments containing powdered ingredients which absorb secretions, for example metal oxides, such as titanium dioxide or zinc oxide, and also tallow and/or aluminium silicates which bind the moisture or the absorbed secretion.

Foams are liquid oil-in-water emulsions in aerosol form. Hydrocarbon compounds are used, inter alia, for the oil-containing phase, for example paraffin oil, fatty alcohols, such as cetyl alcohol, fatty acid esters, such as isopropylmyristate and/or waxes. Suitable emulsifiers are, inter alia, mixtures of emulsifiers having predominantly hydrophilic properties, for example polyoxyethylenesorbitan fatty acid ester, and also emulsifiers having predominantly lipophilic properties, for example sorbitan fatty acid ester. Commercially available additives are usually additionally employed, for example preservatives.

Gels are, in particular, aqueous solutions or suspensions of active substances in which gel formers are dispersed or swelled, in particular cellulose ethers, such as methyl cellulose, hydroxyethyl cellulose, carboxymethyl cellulose or vegetable hydrocolloids, for example sodium alginate, tragacanth or gum Arabic and polyacrylate thickener systems. The gels for example additionally contain polyalcohols, such as propylene glycol or glycerol as moisturisers and wetting agents, such as polyoxyethylenesobitan fatty acid ester. The gels furthermore contain commercially available preservatives, such as benzyl alcohol, phenethyl alcohol, phenoxyethanol and the like.

The following is a list of examples of body care products of this invention and their ingredients:

| Body care product | Ingredients |
|---|---|
| moisturising cream | vegetable oil, emulsifier, thickener, perfume, water, antioxidant, UV absorbers |
| shampoo | surfactant, emulsifier, preservatives, perfume, antioxidant, UV absorbers |

(continued)

| Body care product | Ingredients |
|---|---|
| toothpaste | cleaning agent, thickener, sweetener, flavor, colorant, antioxidant, water, UV absorbers |
| lip-care stick | vegetable oil, wax, $TiO_2$, antioxidant, UV absorbers |

The present body care products, household products, textiles and fabrics have high stability towards color changes and chemical degradation of the ingredients present in these products. For example, present compositions that comprise a dye and/or a pigment are found to have excellent color stability.

Accordingly, the present invention further pertains to a stabilized composition comprising

(a) a body care product, household product, textile or fabric,

(b) an effective stabilizing amount of one or more compounds selected from the group consisting of dilauryl thiodipropionate, distearyl thiodipropionate, dilauryl dithiodipropionate and distearyl dithiodipropionate, and

(e) a dye and/or pigment.

Dyes and/or pigments according to the present invention are for example:

- inorganic pigments, for example iron oxide (Iron Oxide Red, Iron Oxide Yellow, Iron Oxide Black, etc.), Ultramarines, Chromium Oxide Green or Carbon Black;
- natural or synthetic organic pigments;
- disperse dyes which may be solubilzed in solvents like direct hair dyes of the HC type, for example HC Red No. 3, HC Blue No. 2 and all other hair dyes listed in International Cosmetic Ingredient Dictionary and Handbook, 7th edition 19997) or the dispersion dyes listed in Color Index International or Society of Dyers and Colourists;
- color varnishes, so called lakes (insoluble salts of soluble dyes, like many Ca-, Ba- or Al-salts of anionic dyes);
- soluble anionic or cationic dyes, like acid dyes (anionic), basic dyes (cationic), direct dyes, reactive dyes or solvent dyes, e.g. FD&C and D&C dyes, and dyes listed under annex IV or the European Union Cosmetic Directive.

Generally, for the coloration of household- and body care products all substances are suitable which have an absorption in the visible light of electromagnetic radiation (wavelength of ca. 4000 to 700 nm). The absorption is often caused by the following chromophores: Azo-(mono-, di, tris-, or poly-)stilbene-, carotenoide-, diarylmethan-, triarylmethan-, xanthen-, acridin-, quinoline, methin- (also polymethin-), thiazol-, indamin-, indophenol-, azin-, oxazin, thiazin-, anthraquinone-, indigoid-, phtalocyanine- and further synthetic, natural and/or inorganic chromophores.

The dyes and/or pigments of component (e) are present, for example, in the body care and household products in a concentration of about 5 to about 10000 ppm, based on the total formulation by weight, for example from about 10 to about 5000 ppm, for example from about 100 to about 5000 ppm. For example, the dyes and/or pigments of component (e) are present in the body care and household products in a concentration of about 5, 10, 15, 20, 25, 35, 40, 45 or 50 ppm, based on the total formulation by weight. For example, the dyes and/or pigments of component (e) are present from about 5 to about 5000 ppm in the formulations or compositions of this invention.

The present stabilizer systems are also used in household cleaning and treatment agents, for example in laundry products and fabric softeners, liquid cleansing and scouring agents, liquid and/or solid bleaches, glass detergents, neutral cleaners (all-purpose cleaners), acid household cleaners (bath), bathroom cleaners, for instance in washing, rinsing and dishwashing agents, kitchen and oven cleaners, clear rinsing agents, dishwasher detergents, shoe polishes, polishing waxes, floor detergents and polishes, metal, glass and ceramic cleaners, textile-care products, rug cleaners and carpet shampoos, agents for removing rust, color and stains (stain remover salt), furniture and multipurpose polishes and leather and vinyl dressing agents (leather and vinyl sprays) and solid and liquid air fresheners and household cleaning products containing bleach and/or bleaching agents.

The present invention also concerns home care and fabric care products such as drain cleaners, disinfectant solutions, upholstery cleaners, automotive care products (e.g., to clean and/or polish and protect paint, tires, chrome, vinyl, leather, fabric, rubber, plastic and fabric), degreasers, polishes (glass, wood, leather, plastic, marble, granite, and

tile, etc.), and metal polishes and cleaners. Antioxidants are suitable to protect fragrances in above products as well as in dryer sheets. The present invention also relates to home care products such as candles, gel candles, air fresheners and fragrance oils (for the home).

The stabilizers of the present invention may be employed in fabric treatment that takes place after use of the fabric, referred to as fabric care. Such treatments include laundering, which uses detergents and/or fabric conditioner, and the application of non-detergent based fabric care products, such as spray-on products. When employed in this fashion, the present stabilizers are intended for deposition onto the fabric and used to protect the fabric, colorants and fragrances associated with said these fabrics from environmental damage.

Typical examples of household cleaning and treating agents are:

| Household cleaners/- household treating agents | Ingredients |
|---|---|
| detergent concentrate | surfactant mixture, ethanol, antioxidant, water, UV absorbers, antioxidant |
| shoe polish | wax, wax emulsifier, antioxidant, water, preservative, UV absorbers, antioxidant |
| wax-containing floor cleaning agent | emulsifier, wax, sodium chloride, antioxidant, water, preservative, UV absorbers, antioxidant |

The present stabilizers are for example incorporated by dissolution in an oil phase or alcoholic or water phase, where required at elevated temperature.

The present invention also pertains to a method of stabilizing a body care product, household product, textile or fabric, which comprises incorporating therein or applying thereto one or more compounds of component (b) and, one or more compounds of component (c) and/or, one or more compounds according to component (d).

The present invention also pertains to a method of stabilizing a body care product, household product, textile or fabric, each of which contain a dye, which comprises incorporating therein or applying thereto one or more compounds of component (b) and, one or more compounds of component (c) and/or, one or more compounds according to component (d). The stabilizer systems are very effective towards the stabilization of dyes in the present compositions.

In the case of stabilized fabrics, for example dyed fabrics, the present stabilizers are applied thereto via deposition from for instance detergents, fabric conditioners or non-detergent based fabric care products.

The present fabrics are natural or synthetic, and may be woven or nonwoven.

The textiles of this invention are for example textile fiber materials, for example nitrogen-containing or hydroxy-group-containing fiber materials, for instance textile fiber materials selected from cellulose, silk, wool, synthetic polyamides, leather and polyurethanes. Included are cotten, linen and hemp, pulp and regenerated cellulose. Included also are cellulosic blends, for example mixtures of cotton and polyamide or cotton/polyester blends.

The additives of the present invention are for example applied to textiles in a dyeing or printing process, or in a finishing process. For instance, the additives may be applied as part of a dye formulation. The additives may be applied to textiles for example in an ink-jet printing process. The additives are for example applied as part of an aqueous dye solution or printing paste. They may be applied in an exhaust method or dyeing by the padder dyeing method, in which the textiles are impregnated with aqueous dye solutions, which may contain salts, and the dyes and additives are fixed, after an alkali treatment or in the presence of alkali, if appropriate with the action of heat or by storage at room temperature for several hours. After fixing, the dyeings or prints are rinsed thoroughly with cold and hot water, if appropriate with the addition of an agent which has a dispersing action and promotes diffusion of the non-fixed portions.

The dye or ink formulations for application to textiles may comprise further customary additves, for example surfactants, antifoams, antimicrobials and the like, for example as disclosed in U.S. Pat. Nos. 6,281,339, 6,353,094 and 6,323,327.

The following examples describe certain embodiments of this invention, but the invention is not limited thereto. It should be understood that numerous changes to the disclosed embodiments could be made in accordance with the disclosure herein without departing from the spirit or scope of the invention. These examples are therefore not meant to limit the scope of the invention. Rather, the scope of the invention is to be determined only by the appended claims and their equivalents. In these examples all parts given are by weight unless otherwise indicated.

Example 1: Dilauryl Thiodipropionate

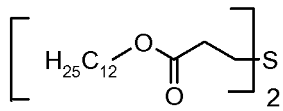

Dilauryl thiodipropionate is a compound known commercially as Irganox PS 800 and is available from Ciba.

Example 2: Vegetable Derived Soap

A vegetable derived soap base is colored with 0.001% Acid Red 33 (D&C Red No. 33) by weight and a fragrance is added (1% by weight of a fruit/berry type). Different known compounds are added at 0.05% by weight and these mixtures are homogenized, extruded, cut and pressed into bar soap samples. From each recipe, a coloristic reading is taken and then samples are stored separately at room temperature (RT) and at 40C. After 3 weeks coloristic readings are taken again and compared to the original data. The results are expressed as Delta E (DE), a measurement for discoloration, and is calculated according to the following equation:

$$\text{Delta E} = [(L_f - L_i)^2 + (a_f - a_i)^2 + (b_f - b_i)^2]^{1/2}$$

f = final reading after weathering
i = initial reading before weathering

The following compounds are evaluated:

Sample A: no stabilizer
Sample B: 0.05% Pentaerythrityl Tetra-di-t-butyl Hydroxyhydrocinnamate
Sample C: 0.05% Octadecyl Di-t-butyl-4-hydroxyhydrocinnamate
Sample D: 0.05% Instant Example 1

Results after 21 days at RT (room temperature) in the dark:

| Sample | DE |
|---|---|
| A | 2.10 |
| B | 1.10 |
| C | 1.67 |
| D | 1.29 |

The unstabilized sample A discolored during storage at room temperature. The results indicate that all tested compounds decrease discoloration, with sample B and sample D, the compound according to instant invention, giving the best results.

Results after 21 days at 40C in the dark:

| Sample | DE |
|---|---|
| A | 4.93 |
| B | 3.29 |
| C | 4.23 |
| D | 3.21 |

After storage at 40C, the unstabilized sample discolored significantly. Instant Example 1, according to the instant invention (sample D), decreased discoloration best, compared to compounds known in the art, B and C.

Example 3: Deodorant Containing Fragrance Formulation

Some fragrance compounds do react with state-of-the-art phenolic antioxidants to form yellowish to red by-products. This makes it impossible to use antioxidants containing phenolic groups, like BHT, or Pentaerythrityl Tetra-di-t-butyl Hydroxyhydrocinnamate, to stabilize such products.

The state of the art antioxidants are tested in such a deodorant formulation, and are compared to the Instant Example 1 according to this invention. For this, 3% by weight of each test compound is added to the fragrance, and then this combination is combined with the deodorant formulation.

Sample A: 3% Pentaerythrityl Tetra-di-t-butyl Hydroxyhydrocinnamate
Sample B: 3% Octadecyl Di-t-butyl-4-hydroxyhydrocinnamate
Sample C: 3% Tetrabutyl Ethylidinebisphenol
Sample D: 3% Instant Example 1

After 4 weeks at 50C, the following results are obtained:

Results after 4 weeks at 50C in the dark:

| Sample | Observation |
|---|---|
| A | dark orange |
| B | Orange |
| C | Yellow |
| D | colorless |

All phenolic-type antioxidants failed to stabilize the instant formulation. The stabilizer according to the instant invention, being "phenol free", did stabilize the formulation from discoloration.

Example 4: Leather Cleaning Formulation

Instant Example 1 is predissolved in the terpene. The components are then stirred in the cited sequence at about 65°C until homogeneous. The mixture is then cooled to room temperature.

| Ingredients | (w/w) % |
|---|---|
| synthetic soap (Zetesap 813) | 7.85 |
| Glycerol | 6.00 |
| anionic surfactant (Lumorol 4192; Mulsifan RT 13) | 22.00 |
| Vaseline | 11.00 |
| paraffin 52/54 | 20.00 |
| Talcum | 2.00 |
| orange terpene | 4.00 |
| Instant Example 1 | 0.02 |
| Water | 27.13 |

Excellent results are achieved for this example of a leather dressing and cleaning agent composition. Upon storage at 40C for 1 month, the formulation performance is better than the same formulation stabilized with state-of-the-art phenolic antioxidant BHT.

Example 5: An Emulsion with Natural Oils

An emulsion with natural oil(s) is prepared comprising the list of ingredients below.

| Phase | Ingredients | (w/w) % |
|---|---|---|
| A | passionflower oil | 8 |
| | glyceryl dioleate | 4 |
| | dicapryl ether | 4 |
| | Isopropylisostearate | 4 |
| | Pentaerythrityl Tetra-di-t-butyl Hydroxyhydrocinnamate | 0.02 |
| | Instant Example 1 | 0.05 |
| B | water, demin. | ad. 100 |
| | EDTA | 0.1 |
| C | Carbomer | 0.15 |
| D | sodium hydroxide | 10% |
| | | 0.20 |
| E | perfume; preservative | q.s. |

The components of phase A are thoroughly mixed in a homogenizer for 10 min at 75-80C. The water phase B, likewise heated to 75-80C beforehand, is slowly added and the mixture is homogenized for 1 minute. The mixture is cooled, with stirring, to 40C and then phases C and E are added and the mixture is homogenized for 1 minute. Subsequently, phase D is added and the mixture is homogenized for thirty seconds and cooled, with stirring, to room temperature. The formulation with and without Instant Stabilizer 1 are stored at 40C for one month.

By means of visual und olfactory assessment, it is seen that the instant compounds dilauryl thiodipropionate and distearyl thiodipropionate of the present invention provide excellent color stability in personal care products.

Example 6: Stabilization of Active Ingredient for Skin Whitening Cream (Hydroquinone)

A skin whitening cream formulation is prepared comprising the list of ingredients below.

| Phase | INCI Name | Trade Name | Supplier | Parts |
|---|---|---|---|---|
| A | Self-emulsifying wax | N/A | N/A | 4.00 |
| A | Cetyl Alcohol | Lanette 16 | Cognis | 1.00 |
| A | Glyceryl Stearate | Teqin 4100 | Degussa | 2.50 |
| B | Glycerin | N/A | Merck | 3.00 |
| B | Deionized Water | Deionized Water | N/A | Qs to 100% |
| C | Sodium Acrylates Copolymer and Paraffinium Liquidum (and) PPG-1 Trideceth-6 | Salcare SC 91 | Ciba | 3.00 |
| D | Alcohol | N/A | N/A | 30.00 |
| D | Hydroquinone | N/A | Eastman | 5.00 |
| D | Test Compound | N/A | Ciba | 0.20 |
| E | Fragrance | Fragrance | N/A | qs |
| E | DMDM Hydantoin | Nipa DMDMH | Nipa | qs |

To the above formulation is added separately various state-of-the-art stabilizers at 0.2% to phase D and the samples are stored at 40C for four weeks in the dark.

Sample A: Nothing

(continued)

| | |
|---|---|
| Sample B: | 0.2 weight % Octadecyl Di-t-butyl-4-hydroxyhydrocinnamate |
| Sample C: | 0.2 weight % 3-(3,4-dimethylphenyl)-5,7-di-tert-butyl-benzofuran-2-one |
| Sample D: | 0.2 weight % Instant Example 1 |
| Sample E: | 0.2 weight % 2’,5’-*%LV*-(5-n-hexyloxycarbonyl-2-methyl-pent-2-yl)-hydroquinone |
| Sample F: | 0.2 weight % Tris(Tetramethylhydroxypiperidinol)Citrate |

After 4 weeks at 40C, the following results are obtained:

Results after 4 weeks at 40C in dark:

| Sample | Observation |
|---|---|
| A | Light red |
| B | Light pink |
| C | Almost colorless |
| D | Colorless |
| E | Light yellow |
| F | Red |

The results are compared to a compound according to present invention. According to the results above, some discoloration is visible even with phenolic antioxidants taught in the art, octadecyl di-t-butyl-4-hydroxyhydrocinnamate. However and quite surprisingly, Instant Example 1 according to instant invention is significantly more effective and no discoloration is observed after storage.

Example 7: Distearyl thiodipropionate

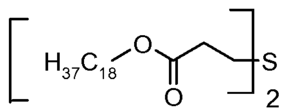

Distearyl thiodipropionate is a compound known commercially.

Example 8: Sprayable Hair Styling Gel

A sprayable hair styling gel formulation is prepared comprising the list of ingredients below.

| Phase | Ingredients | (w/w) % |
|---|---|---|
| A | carbomer (1% dispersion) | 0.30 |
| | water, demin. | 30.00 |
| B | glycerol | 2.00 |
| | methylparaben | 0.20 |
| C | water, demin. | Ad 100 |
| | PVP/VA copolymer | 8.00 |
| | triethanolamine (88%) | 0.12 |
| | EDTA, disodium salt | 0.01 |
| | Instant Example 1 | 0.10 |

Preparation:

The components (A) are dispersed at room temperature.

(B) is mixed under heating until the paraben is completely dissolved and then (B) is added with gentle stirring to (A).

(C) is blended until it is completely dissolved and is slowly added under stirring to the mixture of (A) and (B).

The transparency of the gel can be increased by adding small amounts of triethanolamine (pH=5.6-5.75).

Example 9: Shampoo Formulation

A shampoo formulation is prepared comprising the list of ingredients below.

| Ingredients | (w/w) % |
|---|---|
| cocoamidopropylbetaine | 35.00 |
| water, demin. | Ad.100 |
| citric acid | q.s. (pH) |
| polyquaternium-15 | 0.15 |
| perfume oil | 0.30 |
| chlorophyll | 0.20 |
| Tetrabutyl Ethylidinebisphenol | 0.02 |
| Instant Example 1 | 0.02 |
| colorant (D&C Yellow No.5) | 0.02 |
| sodium chloride | 0.30 |

Preparation: Surfactant and water are blended until a homogeneous solution is obtained. The pH is adjusted to 6.0-6.5 with citric acid and the other components are added in the indicated sequence. The mixture is stirred until it is completely dissolved.

Example 10: A Perfume Formulation

A perfume formulation is prepared comprising the list of ingredients below.

| Ingredients | (w/w) % |
|---|---|
| ethanol, 96% | 60 |
| d-limonene | 5 |
| cedrene | 1.5 |
| citronellol | 0.5 |
| vanillin | 0.5 |
| Benzotriazolyl Dodecyl p-Cresol | 0.05 |
| Tris (Tetramethylhydroxypiperidinol) Citrate | 0.05 |
| Octadecyl Di-t-butyl-4-hydroxyhydrocinnamate | 0.01 |
| Instant Example 1 | 0.01 |
| EDTA, Sodium Salt | 0.01 |
| colorant (D&C Yellow No.5) | 0.1 |
| water | ad. 100 |

Preparation: The components are thoroughly mixed in the indicated sequence at 50C. A clear homogeneous solution is obtained.

Example 11: Green-Colored Glass Detergent Formulation

A green-colored glass detergent formulation is prepared comprising the list of ingredients below.

| Ingredients | (w/w) % |
|---|---|
| anionic / amphoteric surfactants (Lumorol RK) | 0.7 |
| butyl glycol | 5.0 |
| isopropanol | 20.0 |
| d-limonene | 4.00 |
| colorant (D&C Green No.2) | 0.05 |
| Tetrabutyl Ethylidinebisphenol | 0.10 |
| Instant Example 7 | 0.05 |
| water, demin. | ad. 100 |

Preparation: The components are dissolved in the indicated sequence until a clear homogeneous mixture is obtained.

Example 12: Floor Wax Formulation

A floor wax formulation is prepared comprising the list of ingredients below.

| Ingredients | (w/w) % |
|---|---|
| wax mixture | 12 |
| white spirit | ad 100 |
| fragrance | 1.00 |
| Pentaerythrityl Tetra-di-t-butyl Hydroxyhydrocinnamate | 0.02 |
| Bumetrizole | 0.5 |
| Instant Example 1 | 0.02 |

Preparation: The components are stirred in the indicated sequence until a homogeneous mixture is obtained.

Example 13: Lipstick Formulation

A lipstick formulation is prepared comprising the list of ingredients below.

| Ingredients | (w/w) % |
|---|---|
| Carnauba wax | 2.5 |
| Beeswax, white | 20.0 |
| Ozekerite | 10.0 |
| Lanoline, anhydrous | 5.0 |
| Cetyl alcohol | 2.0 |
| Liquid paraffin | 3.0 |
| Isopropyl Myristate | 3.0 |

(continued)

| Ingredients | (w/w) % |
|---|---|
| Propylene glycol recinoleate | 4.0 |
| CI Pigment Red 4 | 9.0 |
| CI Pigment Blue 29 | 1.0 |
| Instant Example 7 | 0.05 |
| Pentaerythrityl Tetra-di-t-butyl Hydroxyhydrocinnamate | 0.05 |
| Castor Oil | ad 100 |

Example 14: Transfer Resistant Lipstick Formulation

A transfer resistant lipstick formulation is prepared comprising the list of ingredients below.

| Ingredients | (w/w) % |
|---|---|
| Cyclomethicone | 41.50 |
| Isodecane | 10.00 |
| D&C Red No. 7 Ca Lake | 8.00 |
| Synthetic wax | 6.00 |
| Isostearyltrimethylpropane siloxysilicate | 5.00 |
| Cetylstearate/acetylated lanolin, 90:10 | 5.00 |
| Ceresin | 4.00 |
| Paraffin | 3.00 |
| Titanium dioxide | 2.00 |
| Methylparaben | 0.30 |
| Propylparaben | 0.10 |
| Instant Example 1 | 0.01 |
| Bumetrizole | 0.10 |

Example 15: Rouge Powder Formulation

A rouge powder formulation is prepared comprising the list of ingredients below.

| Ingredients | (w/w) % |
|---|---|
| Talcum | 56 |
| Zinc Stearate | 15 |
| Rice starch | 15 |
| Iron Oxide Red | 12 |
| Perfume | q.s. |
| Pentaerythrityl Tetra-di-t-butyl Hydroxyhydrocinnamate | 0.02 |
| Instant Example 7 | 0.01 |

Example 16: Foundation Cream Formulation

A foundation cream formulation is prepared comprising the list of ingredients below.

| Ingredients | (w/w) % |
|---|---|
| Titanium dioxide | 12.79 |
| Oleyl alcohol | 4.57 |
| Glyceryl stearate | 3.65 |
| Propylene glycol | 3.65 |
| Stearic acid | 1.83 |
| Magnesium aluminium silicate | 0.91 |
| Triethanolamine 99% | 0.91 |
| Iron Oxide Yellow | 0.64 |
| Iron Oxide Red | 0.32 |
| CI Pigment Brown 6 | 0.37 |
| Carboxymethyl cellulose | 0.10 |
| Instant Example 1 | 0.01 |
| Pentaerythrityl Tetra-di-t-butyl Hydroxyhydrocinnamate | 0.05 |
| Water | ad 100 |

Example 17: Eyeliner Formulation

An eyeliner formulation is prepared comprising the list of ingredients below.

| Ingredients | (w/w) % |
|---|---|
| Polysaccharide resin (Kama KM 13, Kama) | 8.00 |
| Polysaccharide resin (Kama KM 13, Kama) | 8.00 |
| Iron Oxide Black | 6.50 |
| Carnauba wax | 1.00 |
| Triethanolamin, 99% | 1.00 |
| Hydrogenated polyisobutane | 1.00 |
| Hydrogenated polydecene | 1.00 |
| Sorbitan sesquioleate | 1.00 |
| Xanthum gum | 0.50 |
| Carboxymethyl cellulose | 0.40 |
| Magnesium aluminium silicate | 0.40 |
| Methyl paraben | 0.35 |
| Stearic acid | 2.50 |
| Lecithin | 0.20 |
| Imidazolidinyl urea | 0.10 |
| Benzotriazolyl Dodecyl p-Cresol | 0.10 |

| Ingredients | (w/w) % |
|---|---|
| Polysaccharide resin (Kama KM 13, Kama) | 8.00 |
| Instant Example 1 | 0.10 |
| Lactone having the CAS number 216698-07-6 | 0.10 |
| Water | to 100 |

Example 18: Eyelash Makeup Formulation

A eyelash makeup formulation is prepared comprising the list of ingredients below.

| Ingredients | (w/w) % |
|---|---|
| Paraffin Wax | 10.00 |
| Starch | 5.00 |
| Polyethylene | 5.00 |
| Iron Oxide Black | 7.00 |
| Carbomer (Carbopol, BFGoodrich) | 0.50 |
| Hydroxyethylcellulose | 0.50 |
| Panthenol | 2.00 |
| Tetrabutyl Ethylidinebisphenol | 0.01 |
| Lactone having the CAS number 216698-07-6 | 0.01 |
| Instant Example 1 | 0.01 |
| Water | ad 100 |

Example 19. Nail Varnish Formulation

A nail varnish formulation is prepared comprising the list of ingredients below.

| Ingredients | (w/w) % |
|---|---|
| Poly(1-trimethylsilylpropylene) | 0.30 |
| Nitrocellulose | 12.00 |
| Alkyd resin | 10.00 |
| Dibutyl phthalate | 4.00 |
| Camphor | 2.00 |
| Butyl acetate | 49.50 |
| Toluene | 20.00 |
| Pigment Red 57.1 | 1.00 |
| Quaternary bentonite | 1.00 |
| Bumetrizole | 0.50 |
| Instant Example 1 | 0.10 |
| Lactone having the CAS number 216698-07-6 | 0.10 |

Example 20: Generic Shampoo Formulation

A generic shampoo formulation is prepared comprising the list of ingredients below.

| Ingredients | (w/w) % |
|---|---|
| sodium laureth sulfate (30%, TEXAPON NSO, Cognis) | 30% |
| cocamidopropylbetaine (30%, DEHYTON K, Cognis) | 10% |
| colorant* | 0.001% |
| Instant Example 1 | 0.05% |
| Benzophenone-4 | 0.10% |
| Fragrance containing vanillin & Indol | 0.50% |
| citric acid (10% aqueous solution) | to pH 6 |
| deionized water | to 100% |
| *Colorant is PURICOLOR BLUE ABL9 (FD&C Blue No. 1) | |

Example 21: Air Freshener Formulation

An air freshener formulation is prepared comprising the list of ingredients below.

| Ingredients | (w/w) % |
|---|---|
| DME (Propellant) | 30 |
| Polymeric emulsifier | 18 |
| Disodium EDTA | 0.05 |
| Pluronic 10R5 (surfactant) | 1 |
| Triethanolamine | 0.3 |
| Goodrite K752 (Acrylate Polymer) | 0.3 |
| Goodrite 752 | 0.01 |
| Instant Example 1 | 0.02 |
| Pentaerythrityl Tetra-di-t-butyl Hydroxyhydrocinnamate | 0.10 |
| Water | to 100 |

Example 22: Toilet Water Formulation

The components below are thoroughly mixed in the cited sequence at 50C, a clear homogeneous solution being obtained. The UV absorber is, for example, 3-(2H-benzotriazol-2-yl)-4-hydroxy-5-(1-methylpropyl)-benzenesulfonic acid monosodium salt.

| Ingredients | (w/w) % |
|---|---|
| ethanol, 96% | 60 |
| d-limonene | 5 |
| cedrene | 1.5 |
| citronellol | 0.5 |
| savin | 0.5 |
| Instant Example 1 | 0.02 |
| UV absorber | 0.1 |
| S,S-EDDS | 0.005 |
| colorant (D&C Yellow No.5) | 0.02 |

(continued)

| Ingredients | (w/w) % |
|---|---|
| water | ad. 100 |

Excellent results are achieved for this example of a toilet water formulation.

Example 23: Hair Styling Spray Formulation

The hydroxypropyl cellulose is first predissolved in half of the alcohol (Vortex mixer) and is charged with the aminomethylpropanol. The other components - with the exception of the acrylate resin - are dissolved in alcohol and this solution is added, with stirring, to the hydroxypropyl cellulose. Subsequently, the acrylate resin is added and stirred until completely dissolved.

| Ingredients | (w/w) % |
|---|---|
| alcohol, anhydrous | to 100 |
| octylacrylamide/acrylate/butylaminoethylmethacrylate copolymer | 2.52 |
| hydroxypropyl cellulose | 0.51 |
| aminomethylpropanol (95%) | 0.46 |
| Instant Example 7 | 0.02 |
| Tetrabutyl Ethylidinebisphenol | 0.02 |
| perfume oil | 0.20 |

Excellent results are achieved for this example of a hair styling spray formulation.

Example 24: Shampoo Composition for Oily Hair

The components listed below are mixed, with stirring, at room temperature until they are completely dissolved. The pH is 6.5. The UV absorber is, for example, 2-(2-hydroxy-3-dodecyl-5-methylphenyl)-2H-benzotriazole.

| Ingredients | (w/w) % |
|---|---|
| sodium myreth sulfate | 50.00 |
| TEA abietoyl collagen hydrolysate | 3.50 |
| laureth-3 | 3.00 |
| colorant (D&C Red No. 33) | 0.20 |
| Instant Example 1 | 0.05 |
| 4,6-bis(2,4-dimethylphenyl)-2-(4-octyloxy-2-hydroxyphenyl)-s-triazine | 0.15 |
| phosphonomethylchitosan, sodium salt | 0.01 |
| perfume oil | 0.10 |
| water | ad. 100 |

Excellent results are achieved for this example of a shampoo composition for oily hair.

Example 25: Leather Dressing and Cleaning Agent Composition

The stabilizer is predissolved in the terpene. The components are then stirred in the cited sequence at about 65°C until homogeneous. The mixture is then cooled to room temperature.

| Ingredients | (w/w) % |
|---|---|
| synthetic soap (Zetesap 813) | 7.85 |
| Glycerol | 6.00 |
| anionic surfactant (Lumorol 4192; Mulsifan RT 13) | 22.00 |
| Vaseline | 11.00 |
| paraffin 52/54 | 20.00 |
| Talcum | 2.00 |
| orange terpene | 4.00 |
| Instant Example 1 | 0.02 |
| 3-(3,4-dimethylphenyl)-5,7-di-tert-butvl-benzofuran-2-one | 0.02 |
| Water | 27.13 |

Excellent results are achieved for this example of a leather dressing and cleaning agent composition.

Example 26: Glass Detergent Composition

The components listed below are dissolved in the cited sequence until a clear homogeneous mixture is obtained.

| Ingredients | (w/w) % |
|---|---|
| anionic / amphoteric surfactants (Lumorol RK) | 0.7 |
| butyl glycol | 5.0 |
| Isopropanol | 20.0 |
| d-limonene | 4.00 |
| Tetrabutyl Ethylidinebisphenol | 0.03 |
| Instant Example 1 | 0.01 |
| water, demin. | ad. 100 |

Excellent results are achieved for this example of a glass detergent formulation.

Example 27: Protection of Dyes in Fabrics

The instant compounds dilauryl thiodipropionate and distearyl thiodipropionate are each deposited (from water) on a dyed cotton fabric at 0.05, 0.1, 0.2, 0.5 and 1.0 percent by weight, based on the weight of the cotton. The dyed fabrics contain the following dyes at 0.05, 0.1, 0.2 and 0.5 percent by weight based on cotton. This results in 60 separate formulations for each dye listed:

| | | | |
|---|---|---|---|
| Scarlet HE-3G | Crimson HE-XL | Yellow HE-6G | Red HE-XL |
| Blue HE-XL | Turquoise H-A | Navy HE-XL | Remazol |
| Red RB | Brilliant Red RBS | Orange FR | Navy CG |
| Turquoise G | Black B | | |

The cotton fabrics are subjected to light exposure in an Atlas Ci-65 Xenon arc WetherOmeter or to fluorescent lighting. The present stabilizers provide outstanding color protection to the dyed fabrics. This experiment simulates dye

protection achievable through deposition of the present stabilizers via treatment with, for example, stabilizer-containing laundry detergent or fabric conditioner.

Example 28: Protection of Dyes in Fabrics

The instant compounds dilauryl thiodipropionate, distearyl thiodipropionate and UV absorbers, for example 3-(2H-benzotriazol-2-yl)-4-hydroxy-5-(1-methylpropyl)-benzenesulfonic acid monosodium salt, are each deposited (from water) on a dyed cotton fabric at 0.05, 0.1, 0.2, 0.5 and 1.0 percent by weight, based on the weight of the cotton. The dyed fabrics contain the following dyes at 0.05, 0.1, 0.2 and 0.5 percent by weight based on cotton. This results in 60 separate formulations for each dye listed:

| | | | |
|---|---|---|---|
| Scarlet HE-3G | Crimson HE-XL | Yellow HE-6G | Red HE-XL |
| Blue HE-XL | Turquoise H-A | Navy HE-XL | Remazol |
| Red RB | Brilliant Red RBS | Orange FR | Navy CG |
| Turquoise G | Black B | | |

The cotton fabrics are subjected to light exposure in an Atlas Ci-65 Xenon arc WetherOmeter or to fluorescent lighting. The present stabilizers provide outstanding color protection to the dyed fabrics. This experiment simulates dye protection achievable through deposition of the present stabilizers via treatment with for example stabilizer-containing laundry detergent or fabric conditioner.

## Claims

**1.** A stabilized composition comprising

(a) a body care product or household product,
(b) an effective stabilizing amount of at least one compound which is selected from the group consisting of dilauryl thiodipropionate, distearyl thiodipropionate, dilauryl dithiodipropionate and distearyl dithiodipropionate, and
(c) one or more antioxidant(s) selected from the group consisting of formula (1), (2), (3) and (4)

(1) 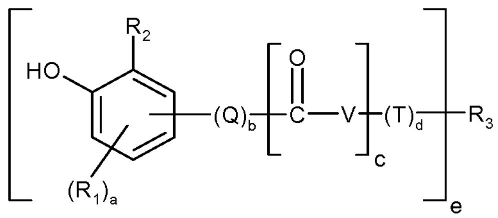 ;

(2) 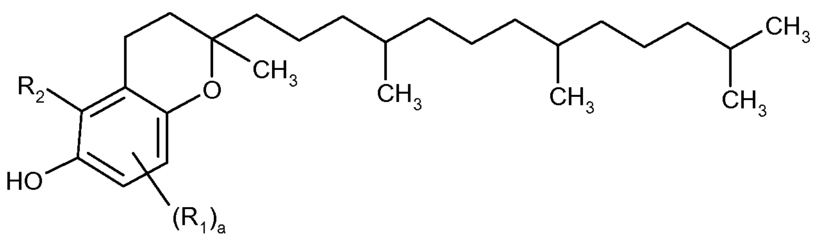 ;

(3) 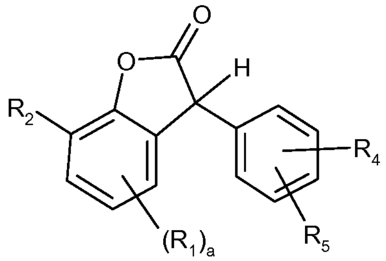 ;

and

(4) 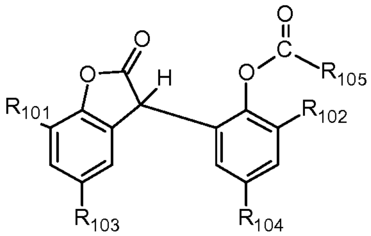 ;

wherein the household product is selected from household cleaning and treating agents,
wherein the household cleaning and treating agents are selected from the group consisting of laundry detergents and fabric softeners, liquid and solid bleach, non-detergent based fabric care products, liquid cleansing and scouring agents, glass detergents, neutral cleaners (all-purpose cleaners), acid household cleaners (bath), bathroom cleaners, washing, rinsing and dishwashing agents, kitchen and oven cleaners, clear rinsing agents, dishwasher detergents, shoe polishes, polishing waxes, floor detergents and polishes, metal, glass and ceramic cleaners, textile-care products, rug cleaners and carpet shampoos, agents for removing rust, color and stains (stain remover salt), furniture and multipurpose polishes and leather and vinyl dressing agents (leather and vinyl sprays) and solid and liquid air fresheners and household cleaning products containing bleach or bleaching agents,
wherein the compounds of component (b), which are selected from the group consisting of lauryl dithiopropionate, distearyl dithiopropionate, dilauryl dithiodipropionate and distearyl dithiodipropionate, are present in a concentration of 10 to 5000 ppm based on the total formulation by weight.
wherein in formula (1), (2), (3) and (4)

$R_1$ is hydrogen; $C_1$-$C_{22}$alkyl; $C_1$-$C_{22}$alkylthio; $C_5$-$C_7$cycloalkyl; phenyl; $C_7$-$C_9$phenylalkyl; or $SO_3M$;
$R_2$ is $C_1$-$C_{22}$alkyl; $C_5$-$C_7$cycloalkyl; phenyl; or $C_7$-$C_9$phenylalkyl;
Q is $-C_mH_{2m}-$;

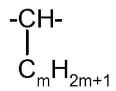 ;

$-C_mH_{2m}-NH$; a radical of formula

(1a) 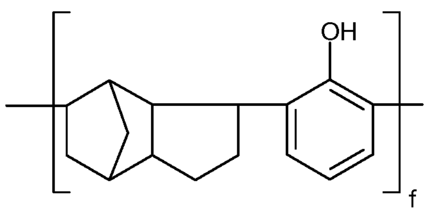 ; or (1b) 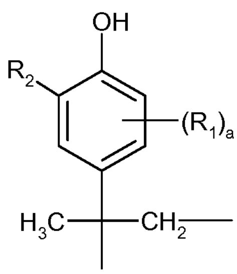

T is $-C_nH_{2n}-$; $-(CH_2)_n-O-CH_2-$;

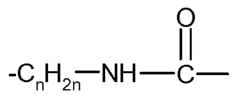 ;

or a radical of formula

(1c) 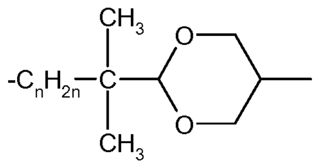 ;

V is -O-; or -NH-;
a is 0; 1; or 2;
b, c and d are each independently of one another 0; or 1;
e is an integer from 1 to 4;
f is an integer from 1 to 3; and
m, n and p are each independently of one another an integer from 1 to 3;
g is 0, 1, 2, or 3;
if e = 1, then
$R_3$ is M; hydrogen; $C_1$-$C_{22}$alkyl; $C_5$-$C_7$cycloalkyl; $C_1$-$C_{22}$alkylthio; $C_2$-$C_{18}$alkenyl; $C_1$-$C_{18}$phenylalkyl; a radical of formula

(1d) 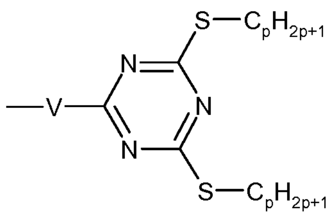 ;

(1e) 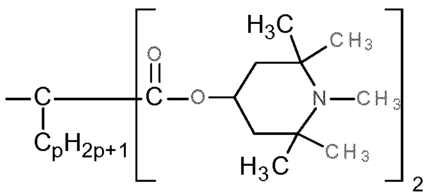 ; or (1f) 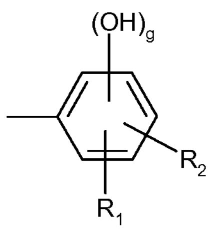

M is alkali; ammonium;
if e = 2, then
$R_3$ is a direct bond; $-CH_2-$;

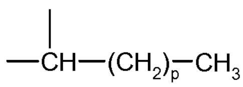 ;

-O-; or -S-;
if e = 3, then
$R_3$ is the radical of formula

(1g) 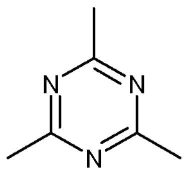 ; (1h) 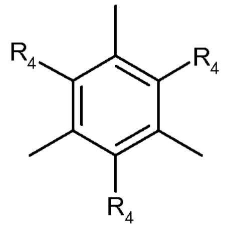 ;

(1i) 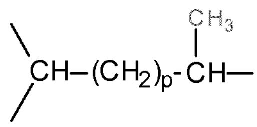 ; or (1k) 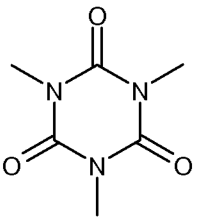 ;

if e = 4, then
R3 is

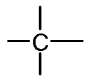 ;

or a direct bond;
$R_4$ and $R_5$ are each independently of the other hydrogen; or $C_1$-$C_{22}$alkyl;
$R_{101}$ and $R_{102}$ are each independently of one another hydrogen; or $C_1$-$C_8$alkyl;
$R_{103}$ and $R_{104}$ are each independently of one another $C_1$-$C_{12}$alkyl; and
$R_{105}$ is $C_1$-$C_7$alkyl.

**2.** A composition according to claim 1 wherein component (b) is selected from the group consisting of dilauryl thiodipropionate and distearyl thiodipropionate.

**3.** A composition according to claim 1 further comprising (d) one or more compounds selected from the group consisting of the ultraviolet light absorbers, hindered amine light stabilizers, complex formers, optical brighteners, surfactants, and polyorganosiloxanes.

**4.** A composition according to claim 1 further comprising (e) a dye.

**5.** A composition according to claim 1 wherein the compounds of component (c) are present in the body care or household products are there in a concentration of 5 to 10000 ppm based on the total formulation by weight.

**6.** A composition according to claim 3 wherein the compounds of component (d) are present in the body care or household products are there in a concentration of 5 to 10000 ppm based on the total formulation by weight.

**7.** A composition according to claim 1 wherein the body care product is selected from the group consisting of skin-care products, bath and shower products, liquid soaps, bar soaps, preparations containing fragrances and odoriferous substances, hair-care products, dentifrices, deodorizing and antiperspirant preparations, decorative preparations, light protection formulations, and preparations containing active ingredients.

**8.** A composition according to claim 7 wherein the skin-care products are selected from the group consisting of body oils, body lotions, body gels, treatment creams, skin protection ointments, shaving preparations, and skin powders.

**9.** A composition according to claim 8 wherein the preparations containing fragrances and olfactory substances are selected from the group consisting of scents, perfumes, toilet waters, and shaving lotions.

**10.** A composition according to claim 6 wherein the hair-care products are selected from the group consisting of shampoos, hair conditioners, 2 in 1 conditioners, leave in and rinse off conditioners, agents for styling and treating hair, perming agents, relaxants, hair sprays and lacquers, hair dyeing systems, permanent, demi-permanent, semi-permanent and temporary hair dyeing systems and hair bleaching agents.

**11.** A composition according to claim 7 wherein the decorative preparations are selected from the group consisting of lipsticks, nail varnishes, eye shadows, mascaras, dry and moist make-up, rouge, powders, depilatory agents, sun care, and after sun products.

**12.** A composition according to claim 7 wherein the preparations containing active ingredients are selected from the group consisting of hormone preparations, vitamin preparations, vegetable extract preparations, and antibacterial preparations.

## Patentansprüche

**1.** Stabilisierte Zusammensetzung, umfassend

(a) ein Körperpflegeprodukt oder Haushaltsprodukt,
(b) eine stabilisierend wirkende Menge mindestens einer Verbindung, die aus der Gruppe bestehend aus Dilaurylthiodipropionat, Distearylthiodipropionat, Dilauryldithiodipropionat und Distearyldithiodipropionat ausgewählt ist, und
(c) ein oder mehrere Antioxidantien aus der Gruppe bestehend aus Formel (1), (2), (3) und (4)

(1) 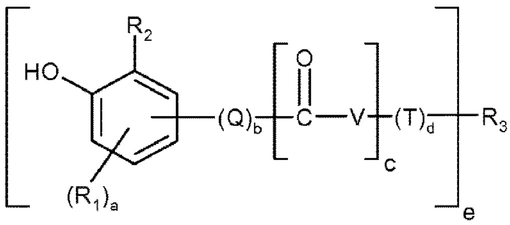 ;

(2) 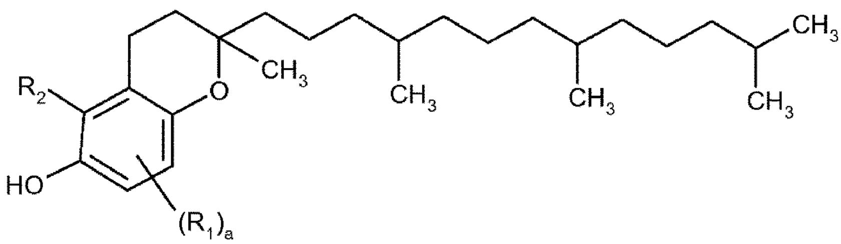 ;

(3) 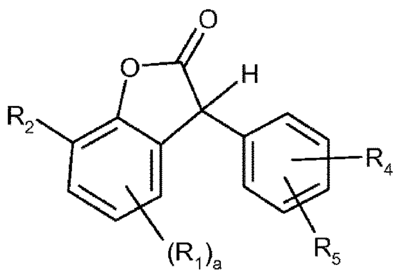

und

(4) 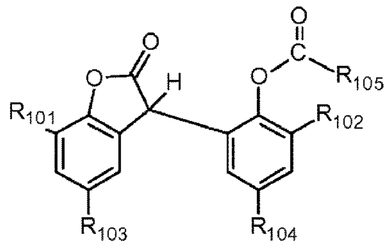 ;

wobei das Haushaltsprodukt aus Haushaltsreinigungsmitteln und -behandlungsmitteln ausgewählt ist, wobei die Haushaltsreinigungsmittel und -behandlungsmittel aus der Gruppe bestehend aus Textilwaschmitteln und Weichspülmitteln, flüssiger und fester Bleiche, nicht reinigungsmittelbasierten Textilpflegeprodukten, flüssigen Reinigungs- und Scheuermitteln, Glasreinigern, Neutralreinigern (Allzweckreinigern), sauren Reinigungsmitteln (Bad), Badreinigern, Wasch-, Spül- und Geschirrspülmitteln, Küchen- und Ofenreinigern, Klarspülern, Spülmaschinenreinigern, Schuhcremes, Polierwachsen, Bodenreinigern und Bohnerwachsen, Metall-, Glas- und Keramikreinigern, Textilpflegeprodukten, Teppichreinigern und Teppichshampoos, Mitteln zur Entfernung von Rost, Farbe und Flecken (Fleckenentfernersalz), Möbel- und Allzweckpolituren und Leder- und Vinyl-Pflegemitteln (Leder- und Vinylsprays) und festen und flüssigen Lufterfrischern und Bleiche oder Bleichmittel enthaltenden Haushaltsreinigungsprodukten ausgewählt sind,
wobei die Verbindungen von Komponente (b), die aus der Gruppe bestehend aus Dilaurylthiodipropionat, Distearylthiodipropionat, Dilauryldithiodipropionat und Distearyldithiodipropionat ausgewählt sind, in einer Konzentration von 10 bis 5000 Gew.-ppm, bezogen auf die gesamte Formulierung, vorliegen,
wobei in Formel (1), (2), (3) und (4)

$R_1$ für Wasserstoff; $C_1$-$C_{22}$-Alkyl; $C_1$-$C_{22}$-Alkylthio; $C_5$-$C_7$-Cycloalkyl; Phenyl; $C_7$-$C_9$-Phenylalkyl oder $SO_3M$ steht;
$R_2$ für $C_1$-$C_{22}$-Alkyl; $C_5$-$C_7$-Cycloalkyl; Phenyl oder $C_7$-$C_9$-Phenylalkyl steht;
Q für $-C_mH_{2m}-$;

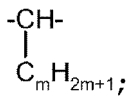

$-C_mH_{2m}-NH$; einen Rest der Formel

(1a) 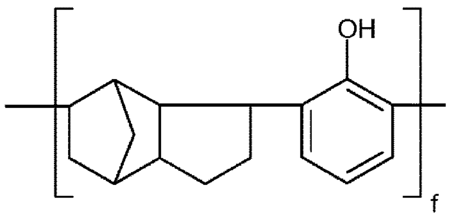

oder

(1b) 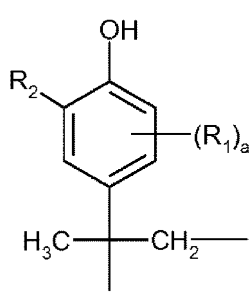

steht;

T für $-C_nH_{2n}-$; $-(CH_2)_n-O-CH_2-$;

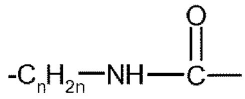

oder einen Rest der Formel (1c)

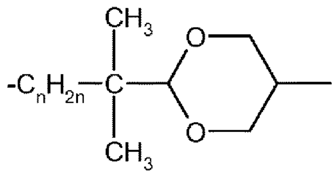

steht;

V für -O- oder -NH- steht;

a für 0; 1 oder 2 steht;

b, c und d jeweils unabhängig voneinander für 0 oder 1 stehen;

e für eine ganze Zahl von 1 bis 4 steht;

f für eine ganze Zahl von 1 bis 3 steht; und

m, n und p jeweils unabhängig voneinander für eine ganze Zahl von 1 bis 3 stehen;

g für 0, 1, 2 oder 3 steht;

dann, wenn e = 1:

$R_3$ für M; Wasserstoff; $C_1$-$C_{22}$-Alkyl; $C_5$-$C_7$-Cycloalkyl; $C_1$-$C_{22}$-Alkylthio; $C_2$-$C_{18}$-Alkenyl; $C_1$-$C_{18}$-Phenylalkyl; einen Rest der Formel

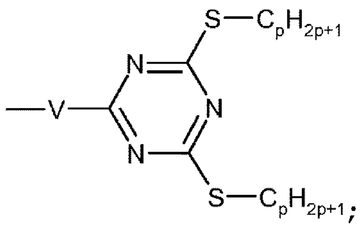

(1d)

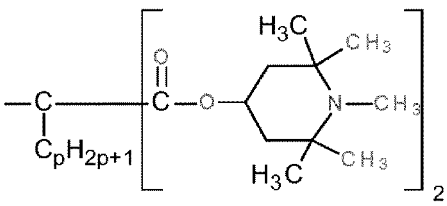

(1e)

oder

(1f)

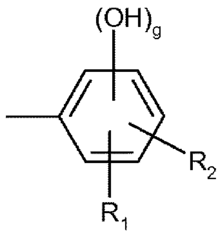

steht;

M für Alkali oder Ammonium steht;

dann, wenn e = 2:

$R_3$ für eine direkte Bindung; $-CH_2-$;

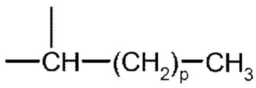;

-O- oder -S- steht;

dann, wenn e = 3:

$R_3$ für den Rest der Formel

(1g) 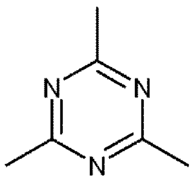;

(1h) 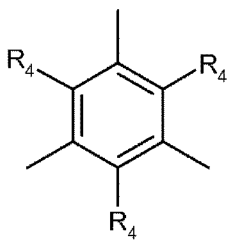; (1i) 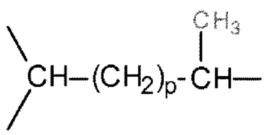

oder

(1k) 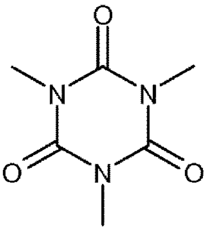 steht;

dann, wenn e = 4:

$R_3$ für

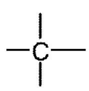

oder eine direkte Bindung steht;

$R_4$ und $R_5$ jeweils unabhängig voneinander für Wasserstoff oder $C_1$-$C_{22}$-Alkyl stehen;

$R_{101}$ und $R_{102}$ jeweils unabhängig voneinander für Wasserstoff oder $C_1$-$C_8$-Alkyl stehen;
$R_{103}$ und $R_{104}$ jeweils unabhängig voneinander für $C_1$-$C_{12}$-Alkyl stehen; und
$R_{105}$ für $C_1$-$C_7$-Alkyl steht.

**2.** Zusammensetzung nach Anspruch 1, wobei Komponente (b) aus der Gruppe bestehend aus Dilaurylthiodipropionat und Distearylthiodipropionat ausgewählt ist.

**3.** Zusammensetzung nach Anspruch 1, ferner umfassend (d) eine oder mehrere Verbindungen aus der Gruppe bestehend aus den Ultraviolettlicht-Absorbern, Lichtschutzmitteln vom Typ der gehinderten Amine, Komplexbildnern, optischen Aufhellern, Tensiden und Polyorganosiloxanen.

**4.** Zusammensetzung nach Anspruch 1, ferner umfassend (e) einen Farbstoff.

**5.** Zusammensetzung nach Anspruch 1, wobei die Verbindungen der Komponente (c) in den Körperpflege- oder Haushaltsprodukten in einer Konzentration von 5 bis 10.000 Gew.-ppm, bezogen auf die gesamte Formulierung, vorliegen.

**6.** Zusammensetzung nach Anspruch 3, wobei die Verbindungen der Komponente (d) in den Körperpflege- oder Haushaltsprodukten in einer Konzentration von 5 bis 10.000 Gew.-ppm, bezogen auf die gesamte Formulierung, vorliegen.

**7.** Zusammensetzung nach Anspruch 1, wobei das Körperpflegeprodukt aus der Gruppe bestehend aus Hautpflegeprodukten, Bade- und Duschprodukten, Flüssigseifen, Stückseifen, Riech- und Geruchsstoffe enthaltenden Zubereitungen, Haarpflegeprodukten, Zahnpflegemitteln, Deodorant- und Antiperspirant-Zubereitungen, dekorativen Zubereitungen, Lichtschutzformulierungen und Wirkstoffe enthaltenden Zubereitungen ausgewählt ist.

**8.** Zusammensetzung nach Anspruch 7, wobei die Hautpflegeprodukte aus der Gruppe bestehend aus Körperölen, Körperlotionen, Körpergelen, Behandlungscremes, Hautpflegesalben, Rasierzubereitungen und Hautpulvern ausgewählt ist.

**9.** Zusammensetzung nach Anspruch 8, wobei die Riech- und Geruchsstoffe enthaltenden Zubereitungen aus der Gruppe bestehend aus Duftstoffen, Parfümen, Eaux de Toilette und Rasierlotionen ausgewählt sind.

**10.** Zusammensetzung nach Anspruch 6, wobei die Haarpflegeprodukte aus der Gruppe bestehend aus Shampoos, Haarkonditionierungsmitteln, 2-in-1-Konditionierungsmitteln, Leave-in- und Rinse-Off-Konditionierungsmitteln, Mitteln zum Frisieren und behandeln von Haar, Dauerwellenmitteln, Entkräuselungsmitteln, Haarsprays und Haarlacken, Haarfärbesystemen, permanenten, demipermanenten, semipermanenten und temporären Haarfärbesystemen und Haarbleichmitteln ausgewählt sind.

**11.** Zusammensetzung nach Anspruch 7, wobei die dekorativen Zubereitungen aus der Gruppe bestehend aus Lippenstiften, Nagellacken, Lidschatten, Wimperntuschen, trockenem und feuchtem Make-up, Rouge, Pudern, Enthaarungsmitteln, Sonnenschutz- und After-Sun-Produkten ausgewählt sind.

**12.** Zusammensetzung nach Anspruch 7, wobei die Wirkstoffe enthaltenden Zubereitungen aus der Gruppe bestehend aus Hormonzubereitungen, Vitaminzubereitungen, Pflanzenextraktzubereitungen und antibakteriellen Zubereitungen ausgewählt sind.

## Revendications

**1.** Composition stabilisée comprenant

(a) un produit de soin corporel ou un produit ménager,
(b) une quantité stabilisante efficace d'au moins un composé qui est choisi dans le groupe constitué des thiodipropionate de dilauryle, thiodipropionate de distéaryle, dithiodipropionate de dilauryle et dithiodipropionate de distéaryle, et
(c) un ou plusieurs antioxydant (s) choisi (s) dans le groupe constitué des formules (1), (2), (3) et (4)

(1) 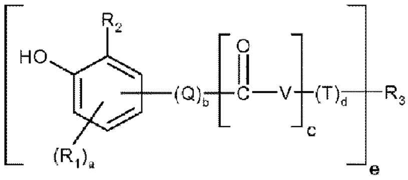 ;

(2) 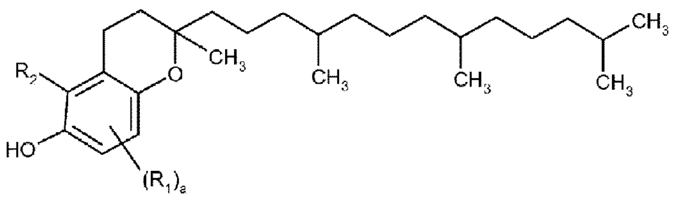 ;

(3) 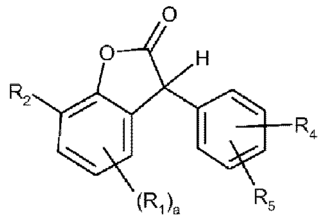 ;

et

(4) 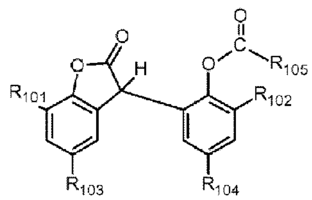 ;

le produit ménager étant choisi parmi des agents de nettoyage et de traitement ménagers, les agents de nettoyage et de traitement ménagers étant choisis dans le groupe constitué de détergents de blanchisserie et d'assouplissants, un javellisant liquide et solide, des produits d'entretien de textile non à base de détergent, des agents de nettoyage et de récurage liquides, des détergents pour verre, des nettoyants neutres (nettoyants tout usage), des nettoyants ménagers acides (bain), des nettoyants pour salle de bain, des agents de lavage, de rinçage et de lavage de la vaisselle, des nettoyants pour cuisine et four, des agents de rinçage limpides, des détergents pour lave-vaisselle, des cirages à chaussures, des cires de polissage, des détergents et produits cirants pour sol, des nettoyants pour métal, verre et céramique, des produits d'entretien de textile, des nettoyants pour carpette et des shampoings pour tapis, des agents pour éliminer la rouille, la coloration et les taches (sel détachant), des produits cirants pour mobilier et multifonctions et des agents d'entretien du cuir et du vinyle (sprays pour cuir et vinyle) et des assainisseurs d'air solides et liquides et des produits nettoyants ménagers contenant un javellisant ou des agents de blanchiment,
dans laquelle les composés de composant (b), qui sont choisis dans le groupe constitué des thiodipropionate de dilauryle, thiodipropionate de distéaryle, dithiodipropionate de dilauryle et dithiodipropionate de distéaryle, sont présents à une concentration de 10 à 5000 ppm sur la base de la formulation totale en poids, dans laquelle, dans les formules (1), (2), (3) et (4)
$R_1$ est hydrogène ; alkyle en $C_1$-$C_{22}$ ; (alkyle en $C_1$-$C_{22}$)thio ; cycloalkyle en $C_5$-$C_7$ ; phényle ; phénylalkyle en $C_7$-$C_9$ ; ou $SO_3M$ ;
$R_2$ est alkyle en $C_1$-$C_{22}$ ; cycloalkyle en $C_5$-$C_7$ ; phényle ; ou phénylalkyle en $C_7$-$C_9$ ;
Q est $-C_mH_{2m}-$ ;

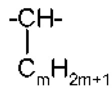 ;

$-C_mH_{2m}-NH$ ; un radical de formule

(1a) 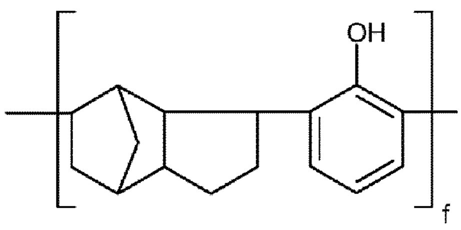 ; ou (1b) 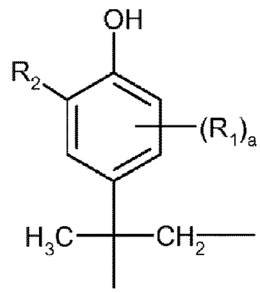

T est $-C_nH_{2n}-$ ; $-(CH_2)_n-O-CH_2-$ ;

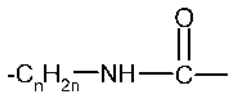 ;

ou un radical de formule

(1c) 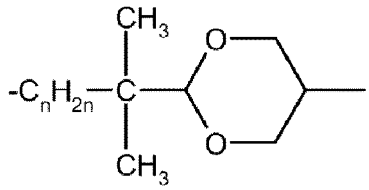 ;

V est -O- ; ou -NH- ;
a est 0 ; 1 ; ou 2 ;
b, c et d sont chacun, indépendamment les uns des autres, 0 ; ou 1 ;
e est un entier de 1 à 4 ;
f est un entier de 1 à 3 ; et
m, n et p sont chacun, indépendamment les uns des autres, un entier de 1 à 3 ;
g est 0, 1, 2 ou 3 ;
si e = 1, alors
$R_3$ est M ; hydrogène ; alkyle en $C_1$-$C_{22}$ ; cycloalkyle en $C_5$-$C_7$ ; (alkyle en $C_1$-$C_{22}$)thio ; alcényle en $C_2$-$C_{18}$ ; phénylalkyle en $C_1$-$C_{18}$ ; un radical de formule

(1d) 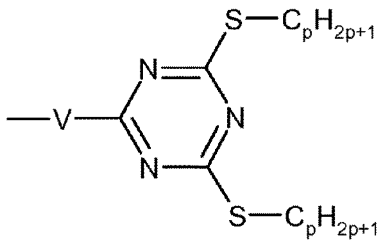 ;

(1e) 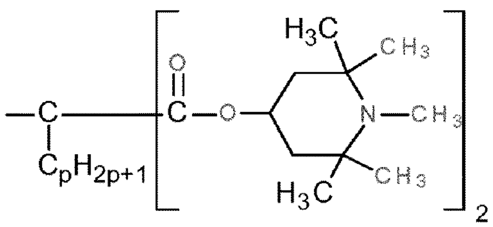 ; ou (1f) 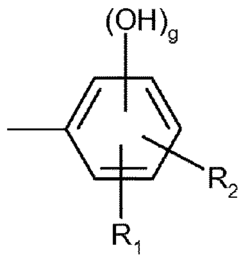

M est un alcali ; ammonium ;
si e = 2, alors
$R_3$ est une liaison directe ; $-CH_2-$ ;

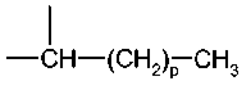 ;

-O- ;
ou -S- ;
si e = 3, alors
$R_3$ est le radical de formule (1g)

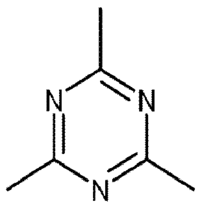 ; (1h) 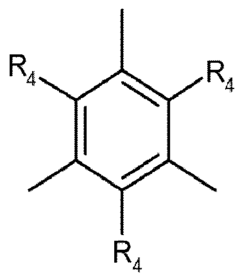 ;

(1i) 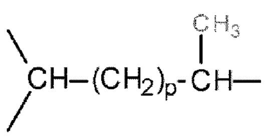 ; ou (1k) 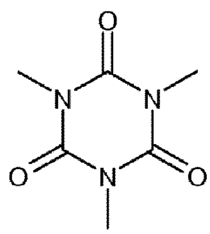 ;

si e = 4, alors
$R_3$ est

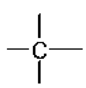 ;

ou une liaison directe ;
$R_4$ et $R_5$ sont chacun, indépendamment l'un de l'autre, hydrogène ; ou alkyle en $C_1$-$C_{22}$ ;
$R_{101}$ et $R_{102}$ sont chacun, indépendamment l'un de l'autre, hydrogène ; ou alkyle en $C_1$-$C_8$ ;
$R_{103}$ et $R_{104}$ sont chacun, indépendamment l'un de l'autre, alkyle en $C_1$-$C_{12}$ ; et
$R_{105}$ est alkyle en $C_1$-$C_7$.

**2.** Composition selon la revendication 1 dans laquelle le composant (b) est choisi dans le groupe constitué du thiodipropionate de dilauryle et du thiodipropionate de distéaryle.

**3.** Composition selon la revendication 1 comprenant en outre (d) un ou plusieurs composés choisis dans le groupe constitué des absorbeurs de lumière ultraviolette, photostabilisants à amine encombrée, agents de formation de complexe, azurants optiques, tensioactifs et polyorganosiloxanes.

**4.** Composition selon la revendication 1, comprenant en outre (e) un colorant.

**5.** Composition selon la revendication 1, dans laquelle les composés du composant (c) sont présents dans les produits de soin corporel ou ménagers à une concentration de 5 à 10 000 ppm sur la base de la formulation totale en poids.

**6.** Composition selon la revendication 3, dans laquelle les composés de composant (d) sont présents dans les produits de soin corporel ou ménagers à une concentration de 5 à 10 000 ppm sur la base de la formulation totale en poids.

**7.** Composition selon la revendication 1, le produit de soin corporel étant choisi dans le groupe constitué de produits de soin pour la peau, produits pour le bain et la douche, savons liquides, savons en barre, préparations contenant des parfums et des substances odorantes, produits de soin capillaire, dentifrices, préparations déodorantes et antitranspirantes, préparations décoratives, formulations de protection contre la lumière et préparations contenant des substances actives.

**8.** Composition selon la revendication 7, les produits de soin pour la peau étant choisis dans le groupe constitué d'huiles pour le corps, lotions pour le corps, gels pour le corps, crèmes de traitement, pommades de protection pour la peau, préparations de rasage et poudres pour la peau.

**9.** Composition selon la revendication 8, les préparations contenant des parfums et des substances odorantes étant choisies dans le groupe constitué de parfums, fragrances, eaux de toilette et lotions de rasage.

**10.** Composition selon la revendication 6, les produits de soin capillaire étant choisis dans le groupe constitué de shampoings, conditionneurs capillaires, conditionneurs 2-en-1, conditionneurs avec et sans rinçage, agents de coiffure et de traitement des cheveux, agents de permanente, agents assouplissants, sprays et laques pour les cheveux, systèmes de coloration capillaire, systèmes de coloration des cheveux permanents, demi-permanents, semi-permanents et temporaires et agents de décoloration des cheveux.

**11.** Composition selon la revendication 7, les préparations décoratives étant choisies dans le groupe constitué de rouges à lèvres, vernis à ongles, ombres à paupières, mascaras, produits de maquillage secs et humides, rouge, poudres, agents dépilatoires, produits de protection solaire et produits après-soleil.

**12.** Composition selon la revendication 7, les préparations contenant des substances actives étant choisies dans le groupe constitué de préparations d'hormones, préparations de vitamines, préparations d'extraits végétaux et préparations antibactériennes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0025730 A
- WO 0025731 A
- US 37738102
- US 60359004
- WO 0107550 A
- US 6254724 B
- WO 2003103622 A
- WO 2006066987 A
- US 20070196290 A
- US 20070196289 A
- US 7264795 B
- US 6919454 B
- US 20070218019 A
- US 2007243147 A
- US 2007243146 A
- US 2007243145 A
- US 09830788 B
- US 09830787 B

### Non-patent literature cited in the description

- International Cosmetic Ingredient Dictionary and Handbook